# EUROPEAN PATENT APPLICATION

(11) **EP 4 715 053 A1**
(43) Date of publication of application: **25.03.2026**
(21) Application number: 24822698.7
(22) Date of filing: 12.06.2024
(51) Int. Cl.: C12N 15/113, A61K 31/713, A61K 47/54, A61P 9/04, A61P 9/12, A61P 13/12

(54) **NUCLEIC ACID TARGETING ANGIOTENSINOGEN AND USE THEREOF**

(30) Priority: 16.06.2023 CN 202310718430
(71) Applicant: SynerK Inc., Grand Cayman KY1-9006 (KY)
(72) Inventor: JIANG, Weiwen, Suzhou, Jiangsu 215123 (CN); YU, Dong, Suzhou, Jiangsu 215123 (CN); LAN, Tao, Suzhou, Jiangsu 215123 (CN)
(74) Representative: Lorenz Seidler Gossel Part. mbB
(86) International application number: PCT/CN2024/098570
(87) International publication number: WO 2024/255749

(57) **Abstract**

A nucleic acid targeting angiotensinogen and a use thereof. The nucleic acid can effectively inhibit the expression of AGT, and can effectively reduce the activation degree of RAAS, so that the nucleic acid can be used for treating and/or preventing diseases related to AGT, such as hypertension.

## Description

### CROSS-REFERENCE TO RELATED APPLICATIONS

The present disclosure claims priority to Chinese Patent Application No. 202310718430.6, filed on June 16, 2023, which is incorporated herein by reference in its entirety.

### TECHNICAL FIELD

The present disclosure pertains to the field of biotechnologies, and specifically, relates to an angiotensinogen-targeting nucleic acid and use thereof.

### BACKGROUND

The renin-angiotensin-aldosterone system (RAAS) is a complex network regulatory system formed by various proteases and short peptides, and serves as a crucial regulatory factor for cardiovascular and renal functions. Excessive activation of the system constitutes a central link in many common pathological conditions, including hypertension, heart failure, and kidney diseases (Lu H et al., Hypertension Res. 39:492-500, 2016). The RAAS pathway initiates with renin cleaving its substrate angiotensinogen to generate the inactive peptide angiotensin I (Ang I), then angiotensin-converting enzyme (ACE) in endothelial cells converts Ang I into angiotensin II (Ang II), with ACE-mediated activation of Ang II being most prevalent in the lungs. Ang II induces vascular contraction and stimulates the adrenal glands to release aldosterone, thereby causing sodium retention and an increase in blood pressure.

RAAS inhibitors include ACE inhibitors, angiotensin II receptor blockers (ARBs), aldosterone antagonists, direct renin inhibitors, and the like. The RAAS inhibitors are existing important medications for treating hypertension and preventing and treating heart failure, and have wide clinical application (Schmieder RE et al., Lancet. 369 (9568):1208-1219; Antonaccio MJ., J Pharmcol., 14:29-45, 1983; Ruiz-Ortega M et al., Trends Cardiovasc Med. 17 (1): 19-25, 2007; Matsubara H., Cric Res. 83 (12):1182-1191, 1998). The ACE inhibitors and angiotensin II receptor blockers can activate compensatory pathways, which further cause reactivation of angiotensin and aldosterone escape. As a result, concentrations of Ang II and aldosterone in the blood return to pre-treatment levels or become even higher (Nobakht N et al., Nat Rev Nephrol. 7:356-359, 2011; Bomback AS and Klemmer PJ., Nat Clin Pract Nephrol. 3:486-492,2007). This may be a key factor causing poor response of patients with refractory hypertension and heart failure to RAAS-inhibiting drugs (Narayan H and Webb DJ., Curr Hypertens Rep. 18:34, 2016, Roig E et al., Eur Heart J. 21: 53-57, 2000). Therefore, a more effective therapeutic strategy should be to target the upstream of RAAS enzymes and receptors to avoid compensatory mechanisms and intracrine pathways that limit therapeutic effects (Mullick AE et al., Hypertension. 70(3): 566-576, 2017).

Angiotensinogen (AGT; also known as SERPINA8, ANHU, and hFLT1) is the common precursor of all angiotensins (Wu C et al., Am J Med Sci. 4: 183-190, 2011; Lu H et al., Hypertens Res. 39: 492-500, 2016). The liver is the main source of AGT in the blood (Yiannilouris G et al., Hypertension. 66: 836-842, 2015; Matsusaka T et al., J Am Soc Nephrol. 23: 1181-1189, 2012). Multiple studies have confirmed that an increase in blood AGT concentration is significantly positively correlated with hypertension (Fasola AF et al., J Appl Physiol. 21:1709-1712, 1966). Reducing the blood AGT concentration can inhibit activity of the RAAS pathway and cause a decrease in blood pressure (Olearczyk J et al., Hypertension Res. 37:405-412, 2014). Intravenous infusion of AGT into rats can increase blood pressure, and the increase in blood pressure can be reversed by treatment with anti-AGT antibodies (Menard J et al., Hypertension. 18:705-707, 1991). AGT-knockout mice have reduced blood pressure, while overexpression of AGT causes an increase in the blood pressure (Kim HS et al., Proc Natl Acad Sci USA92:2735-2739, 1995; Kimura S et al., Embo J. 11:821-827, 1982). Regulating AGT levels to treat hypertension is a promising research and development direction. However, numerous difficulties have been encountered in targeting AGT in conventional methods (Morgan L et al., Int J Biochem Cell Biol.28:1211-1222, 1996).

RNA interference (RNAi) refers to a highly conserved phenomenon of efficient and specific degradation of homologous mRNA induced by double-stranded small interfering RNA (siRNA) in an evolutionary process. Therefore, developing AGT-targeting siRNA is of great significance.

### SUMMARY OF THE INVENTION

An objective of the present disclosure is to overcome the problems existing in the prior art and provide a new AGT-targeting nucleic acid and use thereof.

A first aspect of the present disclosure provides a nucleic acid including a sense strand and an antisense strand, where the sense strand includes a sequence having at least 80% sequence identity with a sequence as set forth in any one of SEQ ID NOs: 1 to 26, and the antisense strand includes a sequence having at least 80% sequence identity with a sequence as set forth in any one of SEQ ID NOs: 27 to 52.

A second aspect of the present disclosure provides a targeted drug delivery system including a targeting moiety, a linking moiety, and the foregoing nucleic acid linked to the targeting moiety through the linking moiety.

A third aspect of the present disclosure provides a pharmaceutical composition including the foregoing nucleic acid or targeted drug delivery system, and a pharmaceutically acceptable carrier.

A fourth aspect of the present disclosure provides a method of inhibiting expression of angiotensin gene in a cell, including: contacting the cell with the foregoing nucleic acid, targeted drug delivery system, or pharmaceutical composition to inhibit the expression of the angiotensin gene in the cell.

A fifth aspect of the present disclosure provides use of the foregoing nucleic acid, targeted drug delivery system, or pharmaceutical composition in any one of the following aspects: (1) treatment and/or prevention of a disease associated with angiotensin; or (2) preparation of a medicament for treating and/or preventing a disease associated with angiotensin.

The inventors of the present disclosure have found that in the renin-angiotensin-aldosterone system, AGT is the most suitable target for RNAi. Therefore, the present disclosure provides a new AGT-targeting nucleic acid and use thereof. The nucleic acid in the present disclosure can effectively inhibit the expression of AGT and reduce the activation level of RAAS, and therefore, can be used to treat and/or prevent hypertension and other AGT-related diseases.

### BRIEF DESCRIPTION OF THE DRAWINGS

To describe the technical solutions in the specific embodiments of the present disclosure or in the prior art more clearly, the following briefly describes the accompanying drawings required for describing the specific embodiments or the prior art. Apparently, the accompanying drawings in the following description show merely some embodiments of the present disclosure, and a person of ordinary skills in the art may still derive other drawings from these accompanying drawings without creative efforts.
FIG. 1 shows the efficacy of SN-682160, SN-682172, SN-682173, SN-682175, and SN-682176 in human AGT transgenic mice in an example of the present disclosure.
FIG. 2 shows the efficacy of SN-682726 and SN-682728 in human AGT transgenic mice in an example of the present disclosure.
FIG. 3 shows the efficacy of SN-682726 and SN-2073 in human AGT transgenic mice in an example of the present disclosure.

### DETAILED DESCRIPTION

The specific embodiments of the present disclosure are described in detail hereinafter. It should be understood that the specific embodiments described herein are only intended to illustrate and explain the present disclosure, rather than limiting the present disclosure. A person skilled in the art can make various modifications and changes to the present disclosure without departing from the scope or spirit of the present disclosure. For example, features illustrated or described as a part of an embodiment can be used in another embodiment to create still another embodiment.

### Term Interpretation

Unless otherwise specified, all terms (including technical and scientific terms) used to disclose the present disclosure have the same meanings as commonly understood by a person of ordinary skill in the art to which the present disclosure belongs. With reference to further guidance, the following definitions are used to better understand teachings in the present disclosure. The terms used herein in the description of the present disclosure are only for the purpose of describing specific examples, and are not intended to limit the present disclosure.

The term "and/or" as used herein refers to a selection range that includes any one of the listed items among two or more related items, as well as any and all combinations of those listed items. These combinations may include any two of the related items, any greater number of the related items, or all of the related items. In the present disclosure, it should be understood that when at least two items are connected by "and/or", the technical solutions undoubtedly include technical solutions connected by logical "AND", and also undoubtedly include technical solutions connected by logical "OR". For example, "A and/or B" includes three parallel solutions: A, B, and both A and B. For another example, technical solutions of "A, B, C and/or D" include any one of A, B, C or D (namely, a technical solution connected by logical "OR"), and also include a combination of any one and all of A, B, C and D, that is, a combination of any two or three of A, B, C and D and a combination of four of A, B, C and D (namely, technical solutions connected by logical "AND").

The terms "comprise", "include", and "contain", as well as their variations, as used herein are synonymous, and are inclusive or open-ended, and do not exclude additional uncited members, elements, or method steps.

In the present disclosure, a numerical range defined by endpoints includes all values and fractions within the range, as well as the cited endpoints.

In the present disclosure, concentration values refer to values that may fluctuate within a certain range. For example, fluctuations may occur within the corresponding precision range. For instance, a value of 2% may allow fluctuations within ±0.1%. For larger values or values that do not require precise control, the meaning may include greater fluctuations. For example, a value of 100 mM may allow fluctuations within ±1%, ±2%, ±5%, etc. For molecular weight, the meaning may include fluctuations within ±10%.

In the present disclosure, descriptions such as "multiple" and "various" refer to two or more than two, unless otherwise specified.

In the present disclosure, technical features described in an open-ended manner encompass both closed technical solutions consisting of the listed features and open technical solutions that include the listed features.

In the present disclosure, expressions such as "preferably", "more preferably", "most preferably", or "desirably" are used solely to describe embodiments or examples with better effects, and shall not be construed as limiting the scope of protection of the invention.

In the present disclosure, terms such as "optionally", "optional", or equivalents thereof indicate that the feature may or may not be present, i.e., selected from two parallel options: presence or absence. Where multiple instances of "optional" features appear in a technical solution, unless otherwise specified and provided there is no contradiction or mutual exclusivity, each "optional" feature shall be considered independently.

In the present disclosure, the term "nucleic acid" refers to a composition containing RNA or RNA-like (for example, chemically modified RNA) oligonucleotide molecules, and the oligonucleotide molecules are capable of degrading or inhibiting (for example, degrading or inhibiting under proper conditions) translation of messenger RNA (mRNA) transcripts of the target mRNA in a sequence-specific manner. The nucleic acid can exert an effect through the RNA interference mechanism (that is, RNA interference induced by interaction via an RNA interference pathway mechanism (RNA-induced silencing complex, RISC) of mammalian cells) or any alternative mechanism or pathway. The scope of lower limitations for the nucleic acids that include a sense strand and an antisense strand and that are disclosed herein includes, but is not limited to, short (or small) interfering RNA (siRNA), double-stranded RNA (dsRNA), microRNA (miRNA), short hairpin RNA (shRNA), and dicer substrates.

In the present disclosure, when referring to the expression of a given gene, the term "silencing", "reduction", "inhibition", "downregulation" or "knockdown" refers to a decrease in gene expression, as measured by the level of RNA transcribed from the gene or the level of polypeptide, protein, or protein subunit translated from the mRNA, in cells, cell populations, tissues, organs, or subjects in which the gene is transcribed, when treated with the nucleic acid described herein, as compared to untreated cells, cell populations, tissues, organs, or subjects.

In the present disclosure, the term "fully complementary" means that, in a hybridized pair of nucleobases or nucleotide sequence molecules, all (100%) bases in a contiguous sequence of the first oligonucleotide hybridize with the same number of bases in a contiguous sequence of the second oligonucleotide. The contiguous sequence may include all or part of the first nucleotide sequence or the second nucleotide sequence.

In the present disclosure, the term "partially complementary" means that, in a hybridized pair of nucleobases or nucleotide sequence molecules, at least 70%, but not all, of the bases in a contiguous sequence of the first oligonucleotide hybridize with the same number of bases in a contiguous sequence of the second oligonucleotide. The contiguous sequence may include all or part of the first nucleotide sequence or the second nucleotide sequence.

In the present disclosure, the term "substantially complementary" means that, in a hybridized pair of nucleobases or nucleotide sequence molecules, at least 85%, but not all, of the bases in a contiguous sequence of the first oligonucleotide hybridize with the same number of bases in a contiguous sequence of the second oligonucleotide. The contiguous sequence may include all or part of the first nucleotide sequence or the second nucleotide sequence.

In the present disclosure, the term "at least partially complementary" means that, in a hybridized pair of nucleobases or nucleotide sequence molecules, the first oligonucleotide and the second oligonucleotide are partially, substantially, or fully complementary.

In the present disclosure, the term "treatment" refers to a method or step taken to relieve or alleviate the number, severity, and/or frequency of one or more disease symptoms in a subject. The treatment may include the prevention, management, prophylactic treatment, and/or inhibition or reduction of the number, severity, and/or frequency of one or more disease symptoms in a subject.

In the present disclosure, the term "link" means that two compounds or molecules are joined through a covalent bond. Unless otherwise specified, as used herein, the term "link" may refer to a connection between a first compound and a second compound with or without any intermediate atom or group of atoms.

### Nucleic Acid

The present disclosure provides a nucleic acid (modified or unmodified) including a sense strand and an antisense strand, the sense strand includes a sequence having at least 80% (for example, 80%, 81%, 82%, 83%, 84%, 85%, 86%, 87%, 88%, 89%, 90%, 91%, 92%, 93%, 94%, 95%, 96%, 97%, 98%, or 99%) sequence identity with the sequence as set forth in any one of SEQ ID NOs: 1 to 26, and the antisense strand includes a sequence having at least 80% (for example, 80%, 81%, 82%, 83%, 84%, 85%, 86%, 87%, 88%, 89%, 90%, 91%, 92%, 93%, 94%, 95%, 96%, 97%, 98%, or 99%) sequence identity with the sequence as set forth in any one of SEQ ID NOs: 27 to 52.

In some embodiments, the antisense strand has 15 to 30 (for example, 15, 16, 17, 18, 19, 20, 21, 22, 23, 24, 25, 26, 27, 28, 29, or 30) nucleotides (bases).

In some embodiments, the sense strand has 15 to 30 (for example, 15, 16, 17, 18, 19, 20, 21, 22, 23, 24, 25, 26, 27, 28, 29, or 30) nucleotides (bases).

During specific implementation, a person skilled in the art can combine the sequences provided in the present disclosure in consideration of the complementarity of the sense strand and the antisense strand, thereby obtaining the combined nucleic acid (siRNA).

In a preferred embodiment of the present disclosure, as shown in Table 1, the nucleic acid is selected from at least one of: siRNA-1 with a sense strand sequence of SEQ ID NO: 1 and an antisense strand sequence of SEQ ID NO: 27, siRNA-2 with a sense strand sequence of SEQ ID NO: 2 and an antisense strand sequence of SEQ ID NO: 28, siRNA-3 with a sense strand sequence of SEQ ID NO: 3 and an antisense strand sequence of SEQ ID NO: 29, siRNA-4 with a sense strand sequence of SEQ ID NO: 4 and an antisense strand sequence of SEQ ID NO: 30, siRNA-5 with a sense strand sequence of SEQ ID NO: 5 and an antisense strand sequence of SEQ ID NO: 31, ..., siRNA-24, siRNA-25, and siRNA-26.

In some preferred embodiments, the antisense strand includes at least 15, 16, 17, 18, 19, 20, 21, 22, or 23 consecutive nucleotides that differ from the sequence as set forth in any one of SEQ ID NOs: 27 to 52 by no more than 0, 1, 2, or 3 nucleotides, and the sense strand includes a nucleotide sequence that is at least partially complementary (for example, partially, substantially, or fully complementary) to the antisense strand.

In some preferred embodiments, the sense strand includes at least 15, 16, 17, 18, 19, 20, or 21 consecutive nucleotides that differ from the sequence as set forth in any one of SEQ ID NOs: 1 to 26 by no more than 0, 1, 2, or 3 nucleotides.

In the present disclosure, the sense strand and the antisense strand may have the same length or different lengths.

All nucleotide groups in the foregoing nucleic acid may be unchemically modified, or may include at least one modified nucleotide group, and the modification may be present in a nucleotide at any position.

In some embodiments, the sense strand and the antisense strand may be partially, substantially, or fully complementary.

In some preferred embodiments, the antisense strand includes a nucleotide sequence that differs from the sequence as set forth in any one of SEQ ID NO: 29, SEQ ID NO: 32, SEQ ID NO: 38, SEQ ID NO: 40, SEQ ID NO: 41, SEQ ID NO: 42, SEQ ID NO: 43, or SEQ ID NO: 44 by 0, 1, or 2 nucleotides, and the sense strand includes a nucleotide sequence that is at least partially complementary (for example, partially, substantially, or fully complementary) to the antisense strand. When the antisense strand has the foregoing sequence, the double-stranded RNA exhibits a significantly better inhibitory effect on AGT.

In some preferred embodiments, the sense strand includes a nucleotide sequence that differs from the sequence as set forth in any one of SEQ ID NO: 3, SEQ ID NO: 6, SEQ ID NO: 12, SEQ ID NO: 14, SEQ ID NO: 15, SEQ ID NO: 16, SEQ ID NO: 17, or SEQ ID NO: 18 by 0, 1, or 2 nucleotides.

In some more preferred embodiments, the antisense strand includes a nucleotide sequence that differs from the sequence as set forth in any one of SEQ ID NO: 29, SEQ ID NO: 40, SEQ ID NO: 41, SEQ ID NO: 43, or SEQ ID NO: 44 by 0, 1, or 2 nucleotides, and the sense strand includes a nucleotide sequence that is at least partially complementary to the antisense strand.

In some preferred embodiments, the sense strand includes a nucleotide sequence that differs from the sequence as set forth in any one of SEQ ID NO: 3, SEQ ID NO: 14, SEQ ID NO: 15, SEQ ID NO: 17, or SEQ ID NO: 18 by 0, 1, or 2 nucleotides.

In some further preferred embodiments, the antisense strand includes a nucleotide sequence that differs from the sequence as set forth in SEQ ID NO: 40 by 0, 1, or 2 nucleotides, and the sense strand includes a nucleotide sequence that is at least partially complementary (for example, partially, substantially, or fully complementary) to the antisense strand. When the antisense strand has this sequence, the double-stranded RNAs with different modifications all exhibit a better inhibitory effect on AGT.

In some preferred embodiments, the sense strand includes a nucleotide sequence that differs from the sequence as set forth in SEQ ID NO: 14 by 0, 1, or 2 nucleotides.

In some preferred embodiments, a content of GC in the nucleic acid is 20%-45%, more preferably 20%-35%.

In some embodiments, when the sense strand or antisense strand of the nucleic acid has a sequence identity of less than 100% with the corresponding sequence according to the present disclosure or differs by one or more nucleotides, the sense strand or antisense strand of the nucleic acid still exhibits an inhibitory effect on AGT that is similar or equivalent to that of the corresponding sequence. For example, the UU linked to the 3' end of the antisense strand of the nucleic acid is replaced with AA, CC, GG, or UG, or a combination of any two nucleic acids. Such nucleic acid sequences also fall within the scope of protection of the present disclosure.

The effect advantages of the technical solutions related to naked sequences (that is, unmodified sequences) according to the present disclosure do not depend on the selection of the modification method or the targeting carrier. The applicable modification solutions and further preferred modification solutions are further introduced in detail as follows:

According to the nucleic acid of the present disclosure, the nucleic acid contains nucleotide groups as basic structural units, the nucleotide group includes a phosphate group, a ribose group, and a base, and preferably, the nucleic acid contains at least one modified nucleotide group. The inhibitory efficiency of the modified nucleic acid on AGT is not less than 50% (for example, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, or 99%).

According to the nucleic acid of the present disclosure, the modified nucleotide group is a nucleotide group with its phosphate group and/or ribose group modified. The modified site(s) may be present at at least 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 of positions 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 of the sense strand and/or the antisense strand.

For example, modification of the phosphate group refers to the modification of oxygen in the phosphate group, including phosphorothioate modification, boranophosphate modification, and the like. As shown in the following formula, oxygen in the phosphate group is replaced with sulfur, borane, amino group, alkyl group, or alkoxy group, respectively. All these modifications can stabilize the structure of the nucleic acid and maintain high specificity and high affinity of base pairing.

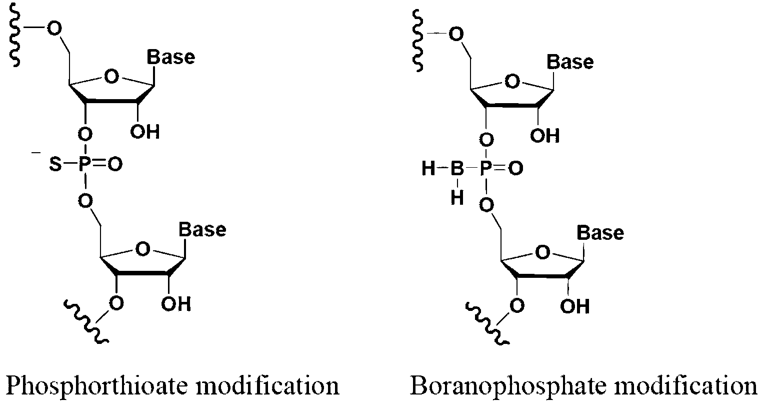

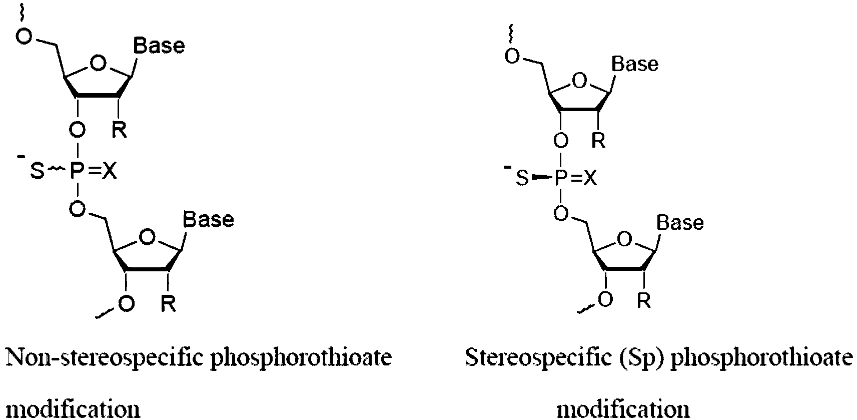

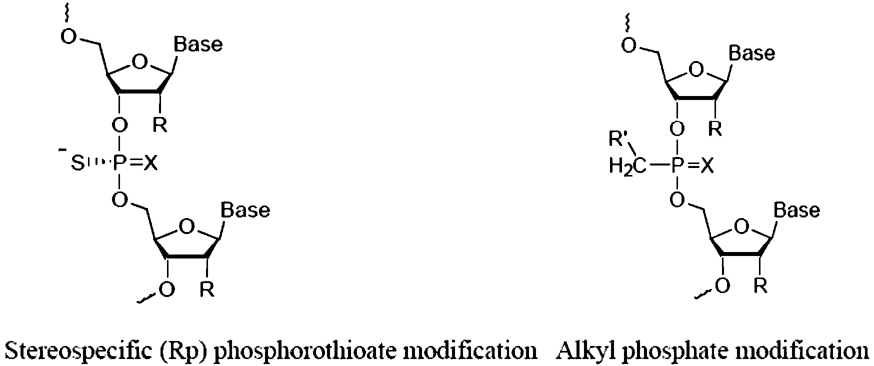

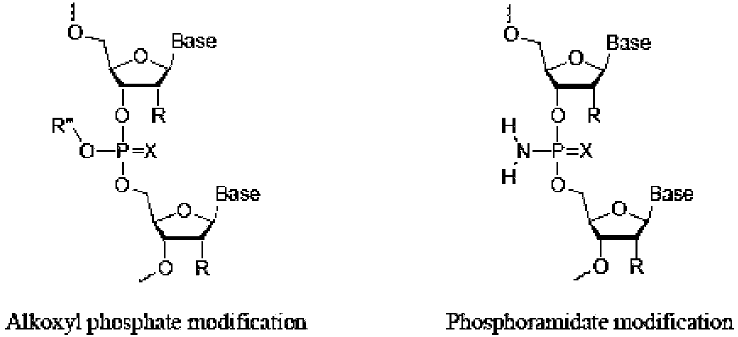

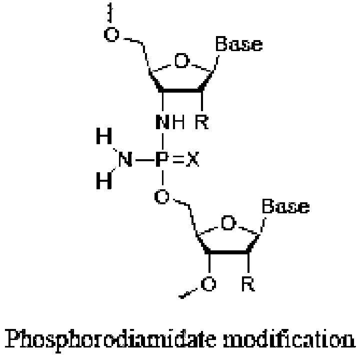

In the above structural formula, Base represents the base A, U, C, G, or T. X may be oxygen (O) or sulfur (S). R may be the same or different in the above structure, such as hydrogen (H), fluorine (F), methoxy (OME) or methoxyethyl (MOE), hydroxyl, allyl, ethylamino, propargyl, amino, cyanoethyl, or acetyl.; R' and R" may each independently be hydrogen (H), methyl (CH₃), ethyl (CH₂CH₃), propyl (CH₂CH₂CH₃), isopropyl (CH(CH3)₂), allyl, propargyl, acyloxybenzyl, or acyloxyethyl.

Modification of the ribose group refers to modification of the 2'-hydroxyl group (2'-OH) in the ribose group. After introducing certain substituents (e.g., a methoxy group or a fluoro group) into the 2'-hydroxyl of the ribose group, the nucleic acid is not readily cleaved by ribonuclease, thereby enhancing stability of the nucleic acid and allowing the nucleic acid to be more resistant to hydrolysis by the nuclease. Modifications of the 2'-hydroxyl group in the nucleotide pentose include 2'-fluoro modification (e.g., 2'-arabino-fluoro modification), 2'-methoxy modification (2'-OME), 2'-methoxyethyl modification (2'-MOE), 2'-2,4-dinitrophenol modification (2'-DNP modification), 2',4'-constrained ethyl modification, 2'-Amino modification, 2'-Deoxy modification, BNA, acyclic nucleic acid modification, mal-positioned nucleic acid modification, L-type nucleic acid modification and the like. BNA (internal ring bridged nucleotide) refers to a constrained or inaccessible nucleotide. BNA may contain a bridging structure of a five-, six-, or seven-membered ring, with a "locked" C3'-endo sugar pucker. The bridge is typically incorporated into the 2'-, 4'-position of the ribose ring to provide the 2',4'-BNA nucleotides such as locked ethyl modification (LNA), constrained ethyl modification (ENA), and constrained ethyl bicyclic nucleic acid modification (cET BNA). Acyclic nucleic acid is a nucleotide formed after the ribose ring of the nucleotide is opened, such as unlocked nucleic acid (UNA) nucleotides and glycerol nucleic acid (GNA) nucleotides. Mal-positioned nucleic acid modification refers to a 3',5'-phosphate bond linkage substituted by the 2',5'-phosphate bond linkage. L-type nucleic acid modification refers to a naturally occurring D-type nucleic acid being substituted by its mirror-stereoscopic counterpart L-type nucleic acid.

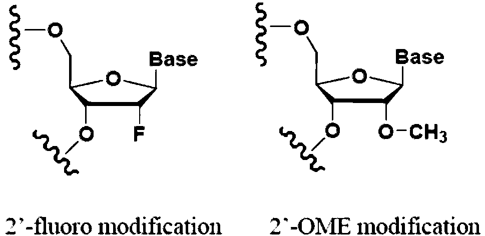

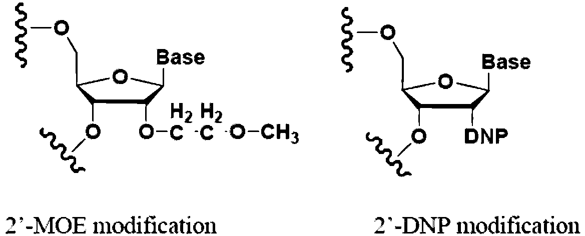

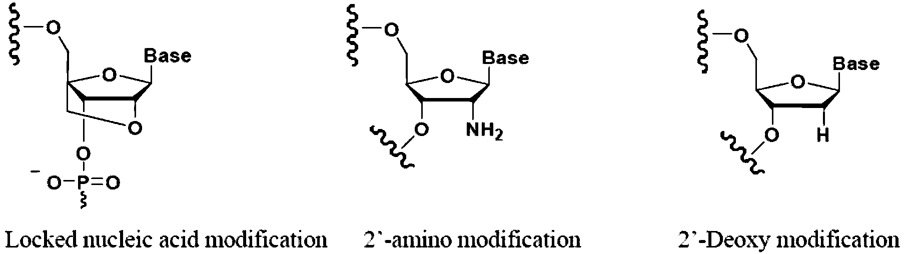

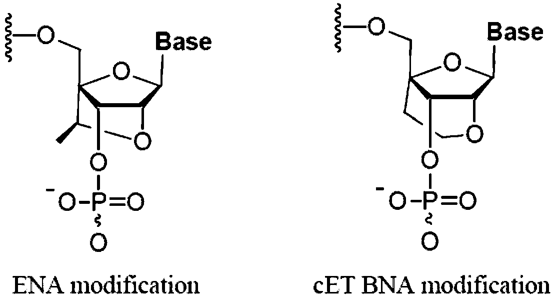

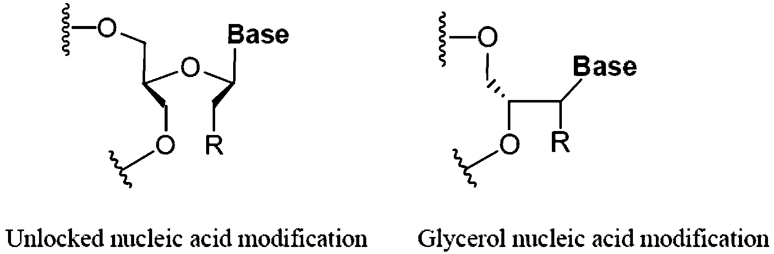

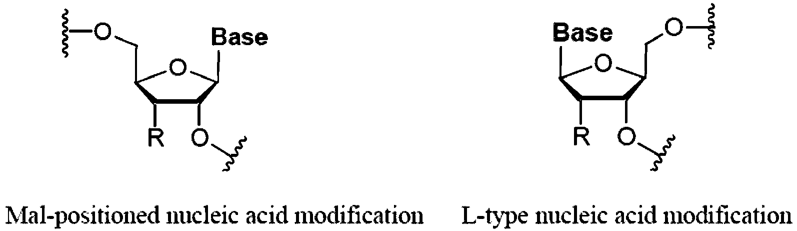

In the above structural formula, Base represents the base A, U, C, G, or T. R may be the same or different in the above structure, such as hydrogen (H), fluorine (F), methoxy (OME) or methoxyethyl (MOE), hydroxyl, allyl, ethylamino, propargyl, cyanoethyl, or acetyl.

According to the nucleic acid of the present disclosure, preferably, the nucleotide group with a modified ribose group is a nucleotide group in which the 2'-OH of the ribose group is replaced with a methoxy or fluoro group.

According to a particularly preferred embodiment of the present disclosure, the nucleotide groups containing uracil bases or cytosine bases in the sense strand of the nucleic acid are the nucleotide groups with their ribose groups modified. That is, 2'-OH of the ribose groups in the nucleotide groups containing uracil bases or cytosine bases in the sense strand of the nucleic acid is replaced with a methoxy or fluoro group. More preferably, the 3' ends of both the sense strand and the antisense strand of the nucleic acid may be linked to dTdT; alternatively, the 3' end of the antisense strand of the nucleic acid may be linked to AA, UU, or a combination of any two nucleotides (which may be, but not limited to, CC, GG, or UG), so that the sequence has specificity as a trigger for mRNA degradation. Nucleic acids with the foregoing modifications exhibit better *in vivo* inhibitory effects, and the foregoing modifications can further reduce the immunogenicity of the nucleic acids in the present disclosure *in vivo.*

The nucleic acid in the present disclosure may further include the modification in which a monophosphate nucleoside is linked to the 5'-end of the antisense strand. The 5'-monophosphate at the end of the guide strand of the siRNA is important for RISC recognition. Phosphorylation of the 5'-hydroxyl group plays a certain role in whether the siRNA can be effectively loaded on the intracellular Ago2. The monophosphate at the 5'-end of the guide strand in the siRNA interacts with Argonaute-2(Ago2) through a hydrogen bond, in order to ensure accurate targeting and precise cleavage of the mRNA target. Several derivatives of the 5'-monophosphate nucleosides are commonly used, this type of derivative of the phosphate nucleoside has been proven to exhibit certain stability in the biological metabolism medium and to play a certain role in facilitating the loading of siRNA guide strand on the intracellular Ago2 *(*Nucleic Acids Research, 2015, 43, 2993-3011). According to the nucleic acid of the present disclosure, preferably, trans-vinyl phosphate (VP) is used as the first choice, and derivatives of nucleoside monophosphate other than those mentioned above may also be included.

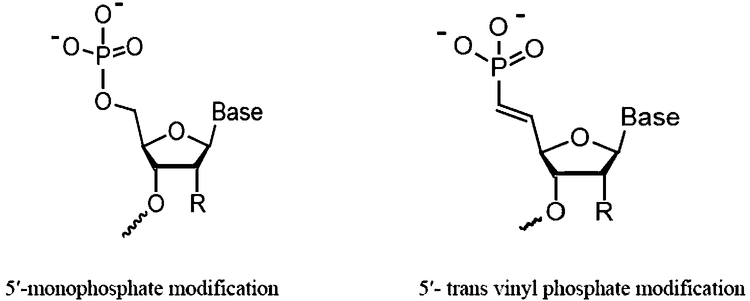

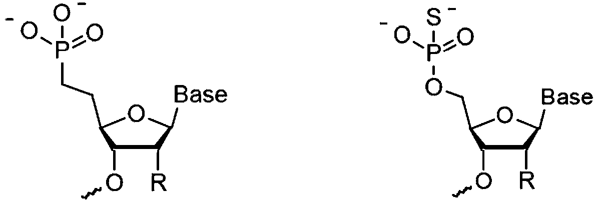

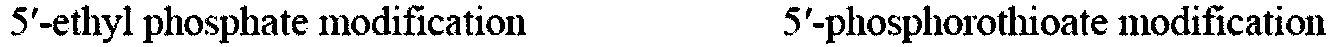

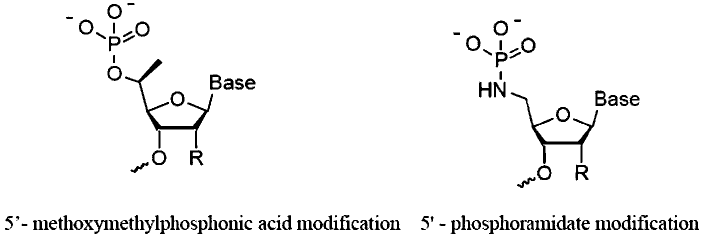

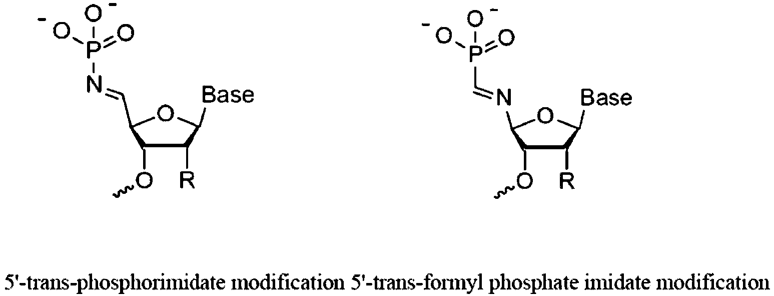

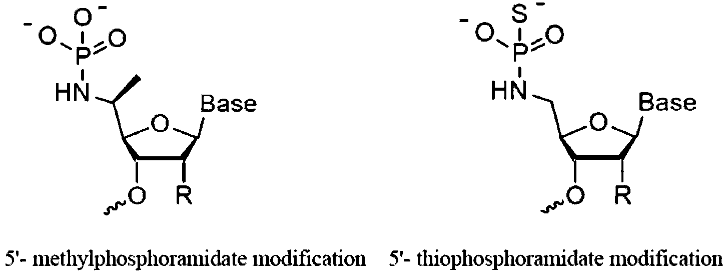

In the above structural formula, Base represents the base A, U, C, G or T. R may be the same or different in the above structure, such as hydrogen (H), fluorine (F), methoxy (OME) or methoxyethyl (MOE), hydroxyl, allyl, ethylamino, propargyl, cyanoethyl, amino, or acetyl.

In some embodiments, at least one nucleotide in the nucleic acid is a modified nucleotide or includes a modified internucleotide linkage.

In some preferred embodiments, the modified nucleotide is one or more selected from 2'-O-methyl nucleotide, 2'-fluoro nucleotide, 2'-deoxy nucleotide, 2',3'-seco nucleotide mimetic, locked nucleotide, 2'-F-arabinonucleotide, 2'-methoxyethyl nucleotide, abasic nucleotide, ribitol, inverted nucleotide, inverted 2'-O-methyl nucleotide, inverted 2'-deoxy nucleotide, 2'-amino modified nucleotide, 2'-alkyl modified nucleotide, morpholino nucleotide, nucleotide containing vinyl phosphonate, nucleotide containing cyclopropyl phosphonate, and 3'-O-methyl nucleotide; and the modified nucleotide is further preferably one or more selected from 2'-O-methyl nucleotide and 2'-fluoro nucleotide.

In some preferred embodiments, the modified internucleotide linkage is preferably one or more selected from phosphorothioate internucleotide linkage and methylphosphonate internucleotide linkage; and the modified internucleotide linkage is further preferably one or more selected from phosphorothioate monoester internucleotide linkage and phosphorothioate diester internucleotide linkage.

In some embodiments, the nucleotides at positions 5 and 6 (counted from the 5' end) of the antisense strand are 2'-O-methyl nucleotides, and the nucleotide at position 7 is 2'-fluoro nucleotide. Based on any sequence according to the present disclosure, the foregoing modifications to the antisense strand can further significantly improve the inhibitory effect of siRNA on AGT.

In some preferred embodiments, the 5' end and 3' end of the antisense strand each contain 2 phosphorothioate internucleotide linkages, nucleotides at positions 1, 2, 7, 9, 14, and 16 (counted from the 5' end) are 2'-fluoro nucleotides, and all of the remaining nucleotides are 2'-O-methyl nucleotides.

In some embodiments, the nucleotide at position 2 (counted from the 5' end) of the sense strand is a 2'-O-methyl nucleotide or a 2'-fluoro nucleotide, preferably a 2'-O-methyl nucleotide. The above modifications to the sense strand can further improve the inhibitory effect of siRNA on AGT.

In some preferred embodiments, the 5' end of the sense strand contains 2 phosphorothioate internucleotide linkages, and the nucleotides at positions 4, 7, 9, 10, and 11 (counted from the 5' end) are 2'-fluoro nucleotides, and all of the remaining nucleotides are 2'-O-methyl nucleotide.

In some embodiments, the antisense strand has at least 15, 16, 17, 18, 19, 20, 21, 22, or 23 consecutive nucleotides that differ from the antisense strand sequence as set forth in any one in Table 3 or Table 8 by no more than 0, 1, 2, or 3 nucleotides; and the sense strand includes at least 15, 16, 17, 18, 19, 20, or 21 consecutive nucleotides that differ from the sense strand sequence as set forth in any one in Table 3 or Table 8 by no more than 0, 1, 2, or 3 nucleotides.

In some embodiments, the sense strand and the antisense strand form an siRNA as set forth in any one in Table 4 or 8.

In some embodiments, the antisense strand includes, from the 5' end to the 3' end, a nucleotide sequence that differs from the following nucleotide sequence by 0, 1, or 2 nucleotides:

AfsCfsacuuUfuUfuguuUfcAfcaaacsasa;
the sense strand includes a nucleotide sequence that is at least partially complementary to the antisense strand.

In some embodiments, the sense strand includes, from the 5' end to the 3' end, a nucleotide sequence that differs from any one of the following nucleotide sequences by 0, 1, or 2 nucleotides:
gsusuUfguGfaAfAfCfaaaaaagugu; or
gsUfsuUfguGfaAfAfCfaaaaaagugu.

In each sequence of the present disclosure, the nucleotide represented by a lowercase letter is a 2'-O-methyl nucleotide; "f" indicates that the adjacent nucleotide on the left side is a 2'-fluoro nucleotide; and "s" indicates that the two adjacent nucleotides on the left and right sides are linked by a phosphorothioate diester bond.

The nucleic acids according to the present disclosure can be obtained in conventional methods in the art, for example solid-phase synthesis and liquid-phase synthesis. Commercially customized services for the solid-phase synthesis are already available, and therefore, can be obtained through commercial purchase. The modified nucleotide groups can be introduced via nucleotide monomers with corresponding modifications.

Based on the foregoing synthesized nucleic acids (siRNA), in the present disclosure, expression plasmids of shRNA that have the same or similar functions as the foregoing siRNA can be further constructed. The methods for constructing such expression plasmids are well-known to a person skilled in the art and are not described in detail herein again.

The present disclosure also provides target gene loci of the foregoing nucleic acids. In some embodiments, the target gene loci are as indicated in any one of the items in Column 1 of Table 1.

**Table 1**

| Target gene loci | SEQ ID NO. | Sense strand (5'-3') | SEQ ID NO. | Antisense strand (5'-3') |
|---|---|---|---|---|
| 778 | 1 | CCACGCUCUCUGGACUUCACA | 27 | UGUGAAGUCCAGAGAGCGUGGGA |
| 977 | 2 | UGGACAACAGCACCUCAGUGU | 28 | ACACUGAGGUGCUGUUGUCCAUU |
| 1145 | 3 | GUCUCACUUUCCAGCAAAACU | 29 | AGUUUUGCUGGAAAGUGAGACUU |
| 1167 | 4 | CCUCAACUGGAUGAAGAAACU | 30 | AGUUUCUUCAUCCAGUUGAGGGA |
| 1450 | 5 | UUCCUGUUUGCUGUGUAUGAU | 31 | AUCAUACACAGCAAACAGGAAUG |
| 1763 | 6 | GCUGGGUUUAUUUUAGAGAAU | 32 | AUUCUCUAAAAUAAACCCAGCUU |
| 1764 | 7 | CUGGGUUUAUUUUAGAGAAUG | 33 | CAUUCUCUAAAAUAAACCCAGUU |
| 1843 | 8 | CCAACCGACCAGCUUGUUUGU | 34 | ACAAACAAGCUGGUCGGUUGGAA |
| 1847 | 9 | CCGACCAGCUUGUUUGUGAAA | 35 | UUUCACAAACAAGCUGGUCGGUU |
| 1849 | 10 | GACCAGCUUGUUUGUGAAACA | 36 | UGUUUCACAAACAAGCUGGUCGG |
| 1849 | 11 | GACCAGCUUGUUUGUGAAACA | 37 | UGUUUCACAAACAAGCUGGUCUU |
| 1851 | 12 | CCAGCUUGUUUGUGAAACAAA | 38 | UUUGUUUCACAAACAAGCUGGUC |
| 1854 | 13 | GCUUGUUUGUGAAACAAAAAA | 39 | UUUUUUGUUUCACAAACAAGCUG |
| 1858 | 14 | GUUUGUGAAACAAAAAAGUGU | 40 | ACACUUUUUUGUUUCACAAACAA |
| 1868 | 15 | CAAAAAAGUGUUCCCUUUUCA | 41 | UGAAAAGGGAACACUUUUUUGUU |
| 1880 | 16 | CCCUUUUCAAGUUGAGAACAA | 42 | UUGUUCUCAACUUGAAAAGGGUU |
| 1893 | 17 | GAGAACAAAAAUUGGGUUUUA | 43 | UAAAACCCAAUUUUUGUUCUCAA |
| 1895 | 18 | GAACAAAAAUUGGGUUUUAAA | 44 | UUUAAAACCCAAUUUUUGUUCUC |
| 1934 | 19 | GCAUUGCCUUCGGUUUGUAUU | 45 | AAUACAAACCGAAGGCAAUGCAA |
| 1935 | 20 | CAUUGCCUUCGGUUUGUAUUU | 46 | AAAUACAAACCGAAGGCAAUGCA |
| 1940 | 21 | CCUUCGGUUUGUAUUUAGUGU | 47 | ACACUAAAUACAAACCGAAGGCA |
| 1945 | 22 | GGUUUGUAUUUAGUGUCUUGA | 48 | UCAAGACACUAAAUACAAACCGA |
| 1950 | 23 | GUAUUUAGUGUCUUGAAUGUA | 49 | UACAUUCAAGACACUAAAUACUU |
| 1959 | 24 | GUGUCUUGAAUGUAAGAACAU | 50 | AUGUUCUUACAUUCAAGACACUU |
| 1961 | 25 | GUCUUGAAUGUAAGAACAUGA | 51 | UCAUGUUCUUACAUUCAAGACAC |
| 2085 | 26 | CCAUAGCUGGUUAUUUCUCCU | 52 | AGGAGAAAUAACCAGCUAUGGUU |

| | | | | |
|---|---|---|---|---|
| Note: Column 1 refers to positions of the first bases of the target genes in the AGT gene sequence, and so on; and the numbers in Columns 2 and 4 represent sequence numbers. For example, "1" represents SEQ ID NO: 1. | | | | |

Coding sequence of human AGT (NM_001382817.3, SEQ ID NO: 53):

1 agaaagtaga ccctcaccag gcatggaatc tgccagtgcc ttgatcttgg acttccagct
61 tccagaactg gtatgcggaa gcgagcaccc cagtctgaga tggctcctgc cggtgtgagc
121 ctgagggcca ccatcctctg cctcctggcc tgggctggcc tggctgcagg tgaccgggtg
181 tacatacacc ccttccacct cgtcatccac aatgagagta cctgtgagca gctggcaaag
241 gccaatgccg ggaagcccaa agaccccacc ttcatacctg ctccaattca ggccaagaca
301 tcccctgtgg atgaaaaggc cctacaggac cagctggtgc tagtcgctgc aaaacttgac
361 accgaagaca agttgagggc cgcaatggtc gggatgctgg ccaacttctt gggcttccgt
421 atatatggca tgcacagtga gctatggggc gtggtccatg gggccaccgt cctctcccca
481 acggctgtct ttggcaccct ggcctctctc tatctgggag ccttggacca cacagctgac
541 aggctacagg caatcctggg tgttccttgg aaggacaaga actgcacctc ccggctggat
601 gcgcacaagg tcctgtctgc cctgcaggct gtacagggcc tgctagtggc ccagggcagg
661 gctgatagcc aggcccagct gctgctgtcc acggtggtgg gcgtgttcac agccccaggc
721 ctgcacctga agcagccgtt tgtgcagggc ctggctctct atacccctgt ggtcctccca
781 cgctctctgg acttcacaga actggatgtt gctgctgaga agattgacag gttcatgcag
841 gctgtgacag gatggaagac tggctgctcc ctgatgggag ccagtgtgga cagcaccctg
901 gctttcaaca cctacgtcca cttccaaggg aagatgaagg gcttctccct gctggccgag
961 ccccaggagt tctgggtgga caacagcacc tcagtgtctg ttcccatgct ctctggcatg
1021 ggcaccttcc agcactggag tgacatccag gacaacttct cggtgactca agtgcccttc
1081 actgagagcg cctgcctgct gctgatccag cctcactatg cctctgacct ggacaaggtg
1141 gagggtctca ctttccagca aaactccctc aactggatga agaaactatc tccccggacc
1201 atccacctga ccatgcccca actggtgctg caaggatctt atgacctgca ggacctgctc
1261 gcccaggctg agctgcccgc cattctgcac accgagctga acctgcaaaa attgagcaat
1321 gaccgcatca gggtggggga ggtgctgaac agcatttttt ttgagcttga agcggatgag
1381 agagagccca cagagtctac ccaacagctt aacaagcctg aggtcttgga ggtgaccctg

1441 aaccgcccat tcctgtttgc tgtgtatgat caaagcgcca ctgccctgca cttcctgggc
1501 cgcgtggcca acccgctgag cacagcatga ggccagggcc ccagaacaca gtgcctggca
1561 aggcctctgc ccctggcctt tgaggcaaag gccagcagca gataacaacc ccggacaaat
1621 cagcgatgtg tcacccccag tctcccacct tttcttctaa tgagtcgact ttgagctgga
1681 aagcagccgt ttctccttgg tctaagtgtg ctgcatggag tgagcagtag aagcctgcag
1741 cggcacaaat gcacctccca gtttgctggg tttattttag agaatggggg tggggaggca
1801 agaaccagtg tttagcgcgg gactactgtt ccaaaaagaa ttccaaccga ccagcttgtt
1861 tgtgaaacaa aaaagtgttc ccttttcaag ttgagaacaa aaattgggtt ttaaaattaa
1921 agtatacatt tttgcattgc cttcggtttg tatttagtgt cttgaatgta agaacatgac
1981 ctccgtgtag tgtctgtaat accttagttt tttccacaga tgcttgtgat ttttgaacaa
2041 tacgtgaaag atgcaagcac ctgaatttct gtttgaatgc ggaaccatag ctggttattt
2101 ctcccttgtg ttagtaataa acgtcttgcc acaataagcc tccaaaaa

### Targeted drug delivery system

The present disclosure further provides a targeted drug delivery system, where the targeted drug delivery system includes a targeting moiety, a linking moiety, and the foregoing nucleic acid linked to the targeting moiety through the linking moiety.

The targeting moiety can further enhance the targeting ability of oligonucleotides, and may be provided by monosaccharides (for example, glucose, mannose, allose, altrose, galactose, galactosamine, N-acetylgalactosamine, talose, fructose, and idose) and/or polypeptides (for example, proteins, monoclonal antibodies, and nanobodies). The linking moiety may be selected from -O-[CH₂CH₂O]ₙ-, -[CH₂]ₘ-CONH-[CH₂]ₙO-, -O-[CH₂CH₂O]ₘ-CONH-[CH₂]ₙO-, and -O-[CH₂]ₘ-CONH-[CH₂H₂O]ₙO-. Herein, m and n may be independently an integer from 1 to 10.

When applied to different targeted drug delivery systems with reference to common knowledge in the art, the nucleic acids (siRNA) in the present disclosure all have better inhibitory effects. In other words, the effects of the naked sequences and modified sequences intended to be protected in the present disclosure do not depend on the selection of the targeting carriers. To further improve the bioavailability and therapeutic efficacy of siRNA, in the present disclosure, the targeted drug delivery system is also optimized to obtain the following technical solutions.

In some embodiments, the linking moiety is linked to the 3' end or 5' end of the sense strand or the antisense strand of the nucleic acid.

In some specific embodiments, the linking moiety is linked to the 3' end of the sense strand of the nucleic acid.

In some embodiments, the targeted drug delivery system includes a ligand and the nucleic acid linked to the ligand.

In some embodiments, the ligand is a GalNAc derivative.

In some embodiments, the ligand is one or more GalNAc derivatives connected via a single-stranded, double-stranded, or triple-stranded branched linker.

In some preferred embodiments, a structure of the targeted drug delivery system is represented by Formula I below:

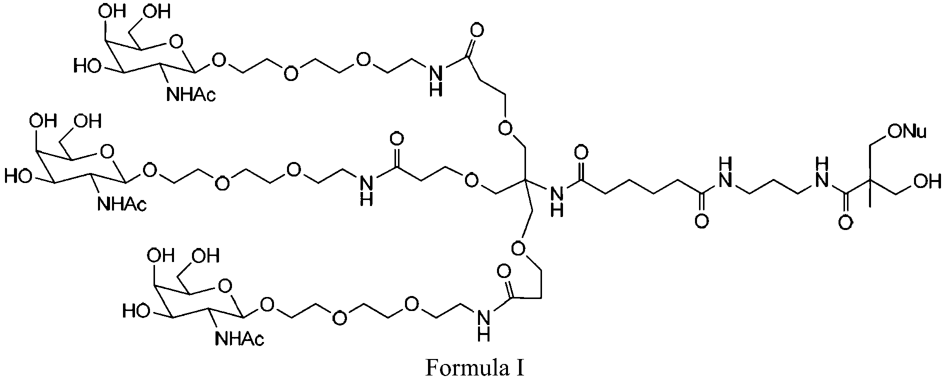

In Formula I, Nu represents the nucleic acid (siRNA). In some embodiments, the compound portion of the system can be linked to the 5' end or 3' end of the sense strand of the siRNA via a phosphodiester bond, or can be linked to the 5' end or 3' end of the antisense strand of the siRNA via a phosphodiester bond. This targeted drug delivery system, with its structural features on the left side, enhances the cellular penetration of nucleic acid drugs (Nu), improves their intracellular stability, and can be prepared through a simple process with strong practical applicability.

### Pharmaceutical Composition

The present disclosure further provides a pharmaceutical composition including the nucleic acid or targeted drug delivery system as described above, and a pharmaceutically acceptable carrier.

The pharmaceutical composition can be prepared from said nucleic acid and the pharmaceutically acceptable carrier using conventional methods. For example, the pharmaceutical composition may be an injection solution. The injection solution can be used for subcutaneous, intramuscular, or intravenous injection.

For the pharmaceutical composition according to the present disclosure, there are no special requirements for the amounts of the nucleic acid, the targeted drug delivery system, or the pharmaceutically acceptable carrier. Generally, relative to 1 part by weight of the nucleic acid (or 1 part by weight of the targeted drug delivery system calculated as per the nucleic acid), the content of the pharmaceutically acceptable carrier can be 1-100,000 parts by weight (for example 1, 5, 10, 50, 100, 500, 1,000, 5,000, 10,000, 50,000, or 100,000 parts by weight, or any value between any two of the above values).

For the pharmaceutical composition according to the present disclosure, the pharmaceutically acceptable carrier may be any of the various carriers conventionally used in the art, including, for example, at least one of pH buffers, protective agents, and osmotic pressure regulators. The pH buffer may be a tris(hydroxymethyl)aminomethane hydrochloride buffer with a pH of 7.5-8.5 and/or a phosphate buffer with a pH of 5.5-8.5, or may be preferably a phosphate buffer with a pH of 5.5-8.5. The protective agent may be at least one of inositol, sorbitol, and sucrose. Based on the total weight of the pharmaceutical composition, the content of the protective agent may be 0.01 wt% to 30 wt% (e.g., 0.01 wt%, 0.05 wt%, 0.1 wt%, 0.5 wt%, 1 wt%, 5 wt%, 10 wt%, 15 wt%, 20 wt%, 25 wt%, or 30 wt%, or any value between any two of the above values). The osmotic pressure regulator may be sodium chloride and/or potassium chloride. The content of the osmotic pressure regulator should be sufficient to ensure that the osmotic pressure of the pharmaceutical composition is 200-700 milliosmoles per kilogram. Based on the required osmotic pressure, a person skilled in the art can determine the content of the osmotic pressure regulator.

According to a preferred embodiment of the present disclosure, the pharmaceutically acceptable carrier is a liposome. The liposome can be any type of liposome capable of encapsulating nucleic acids, and may have a diameter of 25 to 1,000 nm, and include, but not limited to, cholesterol and its analogs or derivatives.

The dosage of the pharmaceutical composition of the present disclosure may be conventional in the art, and may be determined based on various parameters, especially the age, weight, and gender of the subject. For example, for female mice aged 3-4 months and weighing 25g to 30g, based on the amount of the nucleic acid in the pharmaceutical composition, the dosage of the pharmaceutical composition may be 0.01-100 mg per kg of body weight, or may be preferably 1-10 mg per kg of body weight.

### Method and Use

The present disclosure further provides a method of inhibiting expression of angiotensin gene in a cell, where the method includes: contacting the cell with the nucleic acid, targeted drug delivery system, or pharmaceutical composition as described above to inhibit the expression of the angiotensin gene in the cell.

In some embodiments, the cell is in a subject such as a human subject, for example, a subject with an angiotensinogen (AGT)-associated disease.

In some embodiments, the cell is located *in vitro.* The method is intended for research or used for constructing animal models.

In some embodiments, contact between the cell and the nucleic acid inhibits the expression of AGT by at least 50%, 60%, 70%, 80%, 90%, or 95% (e.g., compared to the expression level of AGT before the first contact between the cell and the nucleic acid; for example, before administration of a first dose of the nucleic acid to the subject). In some embodiments, inhibiting the expression of AGT reduces the level of AGT protein in the serum sample of the subject by at least 50%, 60%, 70%, 80%, 90%, or 95%, compared to, for example, the expression level of AGT before the first contact between the cell and the nucleic acid.

The present disclosure further provides the use of the nucleic acid, targeted drug delivery system, or pharmaceutical composition as described above in any one of the following aspects: (1) treatment and/or prevention of a disease associated with angiotensin; or (2) preparation of a medicament for treating and/or preventing a disease associated with angiotensin.

In some embodiments, the disease is: (i) a disease associated with enhancement or elevation of an activity level of angiotensin; or (ii) a disease that benefits from reduced expression of angiotensinogen.

In some embodiments, the disease is a disease associated with excessive activation of the renin-angiotensin-aldosterone system (RAAS). In some specific embodiments, "disease associated with excessive activation of the RAAS system" may include hypertension, heart failure, kidney diseases, presenile dementia, ophthalmic diseases, and the like.

In some embodiments, the disease is selected from one or more of hypertension, hypertensive disorder, borderline hypertensive disorder, primary hypertensive disorder, secondary hypertensive disorder, isolated systolic or diastolic hypertensive disorder, pregnancy-related hypertensive disorder, diabetic hypertensive disorder, resistant hypertensive disorder, refractory hypertensive disorder, paroxysmal hypertensive disorder, renovascular hypertensive disorder, Goldblatt hypertension, hypertensive disorder associated with low plasma renin activity or plasma renin concentration, high intraocular pressure, glaucoma, pulmonary arterial hypertension, portal hypertension, systemic venous hypertension, systolic hypertensive disorder, unstable hypertensive disorder; hypertensive heart disease, hypertensive nephropathy, atherosclerosis, arteriosclerosis, vascular disease, diabetic nephropathy, diabetic retinopathy, chronic heart failure, cardiomyopathy, diabetic cardiomyopathy, glomerulosclerosis, coarctation of the aorta, aortic aneurysm, ventricular fibrosis, heart failure, myocardial infarction, angina pectoris, stroke, nephropathy, renal failure, systemic sclerosis, intrauterine growth restriction (IUGR), fetal growth restriction, obesity, hepatic steatosis/fatty liver, non-alcoholic steatohepatitis (NASH), non-alcoholic fatty liver disease (NAFLD), glucose intolerance, type 2 diabetes mellitus (non-insulin-dependent diabetes mellitus), and metabolic syndrome.

In the present disclosure, the subject may be a mammal, including a primate (for example, a human and a non-human primate such as a monkey and a chimpanzee), a non-primate (for example, cattle, a pig, a horse, a goat, a rabbit, sheep, a hamster, a guinea pig, a cat, a dog, a rat, or a mouse), or a bird. In some embodiments, the subject is preferably a primate, more preferably, a human. Administration can be performed via multiple routes, depending on whether local treatment or systemic treatment is required. The administration routes may include, but not limited to, intravenous administration, intraarterial administration, subcutaneous administration, intraperitoneal administration, transdermal administration (for example, via an implanted device), and intra-soft tissue administration. The dosage may refer to the foregoing description. Details are not described again herein.

In some embodiments, the nucleic acid, the targeted drug delivery system, or the pharmaceutical composition is administered to a subject via subcutaneous administration, intravenous administration, and/or intramuscular administration.

### Examples

The embodiments of the present disclosure are described in detail below with reference to examples. It should be understood that these examples are only used to illustrate the present disclosure and are not intended to limit the scope of the present disclosure. For experimental methods in the following examples where specific conditions are not indicated, reference should be made to the guidance provided in the present disclosure. Alternatively, standard laboratory manuals, conventional conditions known in the art, or manufacturer-recommended procedures may be adopted.

In the following specific examples, measurement parameters related to raw material components may exhibit slight deviations within the weighing accuracy range unless otherwise specified. Parameters involving temperature and time may allow acceptable deviations resulting from instrument testing accuracy or operational precision.

### Example 1 Sequence Screening of siRNA

Double-stranded siRNAs can enter cells and be incorporated into the RISC complex. In the RISC complex, the double-stranded siRNA is unwound by the endonuclease Ago2, leaving only the antisense strand to recognize the target mRNA. A GC content of 35-55% is generally regarded as the optimal choice for siRNA. GC content is also correlated with the melting temperature of siRNA. However, siRNAs with lower GC content may unwind more rapidly or efficiently due to their thermal stability. The siRNA sequences designed in the present disclosure (as shown in Table 2) include those with lower GC content, thereby facilitating the optimal selection of siRNA.

**Table 2**

| Target gene loci | Sequence (5'-3') | GC% |
|---|---|---|
| 778 | CCACGCUCUCUGGACUUCACA | 57.10% |
| 977 | UGGACAACAGCACCUCAGUGU | 52.40% |
| 1145 | GUCUCACUUUCCAGCAAAACU | 42.90% |
| 1167 | CCUCAACUGGAUGAAGAAACU | 42.90% |
| 1450 | UUCCUGUUUGCUGUGUAUGAU | 38.10% |
| 1763 | GCUGGGUUUAUUUUAGAGAAU | 33.30% |
| 1764 | CUGGGUUUAUUUUAGAGAAUG | 33.30% |
| 1843 | CCAACCGACCAGCUUGUUUGU | 52.40% |
| 1847 | CCGACCAGCUUGUUUGUGAAA | 47.60% |
| 1849 | GACCAGCUUGUUUGUGAAACA | 42.90% |
| 1849A | GACCAGCUUGUUUGUGAAACA | 42.90% |
| 1851 | CCAGCUUGUUUGUGAAACAAA | 38.10% |
| 1854 | GCUUGUUUGUGAAACAAAAAA | 28.60% |
| 1858 | GUUUGUGAAACAAAAAAGUGU | 28.60% |
| 1868 | CAAAAAAGUGUUCCCUUUUCA | 33.30% |
| 1880 | CCCUUUUCAAGUUGAGAACAA | 38.10% |
| 1893 | GAGAACAAAAAUUGGGUUUUA | 28.60% |
| 1895 | GAACAAAAAUUGGGUUUUAAA | 23.80% |
| 1934 | GCAUUGCCUUCGGUUUGUAUU | 42.90% |
| 1935 | CAUUGCCUUCGGUUUGUAUUU | 38.10% |
| 1940 | CCUUCGGUUUGUAUUUAGUGU | 38.10% |
| 1945 | GGUUUGUAUUUAGUGUCUUGA | 33.30% |
| 1950 | GUAUUUAGUGUCUUGAAUGUA | 28.60% |
| 1959 | GUGUCUUGAAUGUAAGAACAU | 33.30% |
| 1961 | GUCUUGAAUGUAAGAACAUGA | 33.30% |
| 2085 | CCAUAGCUGGUUAUUUCUCCU | 42.90% |

A preferred sequence is GUUUGUGAAACAAAAAAGUGU, with a GC content of 28.6%.

The sense strand and antisense strand sequences corresponding to the foregoing sequences are shown in Table 1.

In this example, each sense strand and antisense strand were further modified. The modified sequences are shown in Table 3.

**Table 3**

| No. | Sense strand (5'-3') | No. | Antisense strand (5'-3') |
|---|---|---|---|
| SN-22158 | cscsacgcUfcUfCfUfggacuucaca | SN-52158 | usGfsuGfaAfguccaGfaGfagcguggsgsa |
| SN-22159 | usgsgacaAfcAfGfCfaccucagugu | SN-52159 | asCfsaCfuGfaggugcuGfuUfguccasusu |
| SN-22160 | gsuscucaCfuUfUfCfcagcaaaacu | SN-52160 | asGfsuUfuUfgcuggaaAfgUfgagacsusu |
| SN-22161 | cscsucaaCfuGfGfAfugaagaaacu | SN-52161 | asGfsuUfuCfuucauccAfgUfugaggsgsa |
| SN-22163 | ususccugUfuUfGfCfuguguaugau | SN-52163 | asUfscAfuAfcacagcaAfaCfaggaasusg |
| SN-22164 | gscsugggUfuUfAfUfuuuagagaau | SN-52164 | asUfsuCfuCfuaaaauaAfaCfccagcsusu |
| SN-22165 | csusggguUfuAfUfUfuuagagaaug | SN-52165 | csAfsuUfcUfcuaaaauAfaAfcccagsusu |
| SN-22166 | cscsaaccGfaCfCfAfgcuuguuugu | SN-52166 | asCfsaAfaCfaagcuggUfcGfguuggsasa |
| SN-22167 | cscsgaccAfgCfUfUfguuugugaaa | SN-52167 | usUfsuCfaCfaaacaagCfuGfgucggsusu |
| SN-22168 | gsasccagCfuUfGfUfuugugaaaca | SN-52168 | usGfsuUfuCfacaaacaAfgCfuggucsgsg |
| SN-22169 | gsasccagCfuUfGfUfuugugaaaca | SN-52169 | usGfsuUfuCfacaaacaAfgCfuggucsusu |
| SN-22170 | cscsagcuUfgUfUfUfgugaaacaaa | SN-52170 | usUfsuGfuUfucacaaaCfaAfgcuggsusc |
| SN-22171 | gscsuuguUfuGfUfGfaaacaaaaaa | SN-52171 | usUfsuUfuUfguuucacAfaAfcaagcsusg |
| SN-22172 | gsusuuguGfaAfAfCfaaaaaagugu | SN-52172 | asCfsaCfuUfuuuuguuUfcAfcaaacsasa |
| SN-22173 | csasaaaaAfgUfGfUfucccuuuuca | SN-52173 | usGfsaAfaAfgggaacaCfuUfuuuugsusu |
| SN-22174 | cscscuuuUfcAfAfGfuugagaacaa | SN-52174 | usUfsgUfuCfucaacuuGfaAfaagggsusu |
| SN-22175 | gsasgaacAfaAfAfAfuuggguuuua | SN-52175 | usAfsaAfaCfccaauuuUfuGfuucucsasa |
| SN-22176 | gsasacaaAfaAfUfUfggguuuuaaa | SN-52176 | usUfsuAfaAfacccaauUfuUfuguucsusc |
| SN-22177 | gscsauugCfcUfUfCfgguuuguauu | SN-52177 | asAfsuAfcAfaaccgaaGfgCfaaugcsasa |
| SN-22178 | csasuugcCfuUfCfGfguuuguauuu | SN-52178 | asAfsaUfaCfaaaccgaAfgGfcaaugscsa |
| SN-22179 | escsuucgGfuUfUfGfuauuuagugu | SN-52179 | asCfsaCfuAfaauacaaAfcCfgaaggscsa |
| SN-22180 | gsgsuuugUfaUfUfUfagugucuuga | SN-52180 | usCfsaAfgAfcacuaaaUfaCfaaaccsgsa |
| SN-22181 | gsusauuuAfgUfGfUfcuugaaugua | SN-52181 | usAfscAfuUfcaagacaCfuAfaauacsusu |
| SN-22182 | gsusgucuUfgAfAfUfguaagaacau | SN-52182 | asUfsgUfuCfuuacauuCfaAfgacacsusu |
| SN-22183 | gsuscuugAfaUfGfUfaagaacauga | SN-52183 | usCfsaUfgUfucuuacaUfuCfaagacsasc |
| SN-22184 | cscsauagCfuGfGfUfuauuucuccu | SN-52184 | asGfsgAfgAfaauaaccAfgCfuauggsusu |

In the table, a/c/g/u = 2'-OMe nucleotide; Af/Cf/Gf/Uf = 2'-F nucleotide; and s = phosphorothioate diester bond.

The sense strands and antisense strands in Table 2 formed the modified double-stranded siRNAs in Table 4 via solid-phase synthesis.

**Table 4**

| No. | Sense strand (5'-3') | Antisense strand (5'-3') |
|---|---|---|
| SN-252158 | cscsacgcUfcUfCfUfggacuucaca | usGfsuGfaAfguccaGfaGfagcguggsgsa |
| SN-252159 | usgsgacaAfcAfGfCfaccucagugu | asCfsaCfuGfaggugcuGfuUfguccasusu |
| SN-252160 | gsuscucaCfuUfUfCfcagcaaaacu | asGfsuUfuUfgcuggaaAfgUfgagacsusu |
| SN-252161 | cscsucaaCfuGfGfAfugaagaaacu | asGfsuUfuCfuucauccAfgUfugaggsgsa |
| SN-252163 | ususccugUfuUfGfCfuguguaugau | asUfscAfuAfcacagcaAfaCfaggaasusg |
| SN-252164 | gscsugggUfuUfAfUfuuuagagaau | asUfsuCfuCfuaaaauaAfaCfccagcsusu |
| SN-252165 | csusggguUfuAfUfUfuuagagaaug | csAfsuUfcUfcuaaaauAfaAfcccagsusu |
| SN-252166 | cscsaaccGfaCfCfAfgcuuguuugu | asCfsaAfaCfaagcuggUfcGfguuggsasa |
| SN-252167 | cscsgaccAfgCfUfUfguuugugaaa | usUfsuCfaCfaaacaagCfuGfgucggsusu |
| SN-252168 | gsasccagCfuUfGfUfuugugaaaca | usGfsuUfuCfacaaacaAfgCfuggucsgsg |
| SN-252169 | gsasccagCfuUfGfUfuugugaaaca | usGfsuUfuCfacaaacaAfgCfuggucsusu |
| SN-252170 | cscsagcuUfgUfUfUfgugaaacaaa | usUfsuGfuUfucacaaaCfaAfgcuggsusc |
| SN-252171 | gscsuuguUfuGfUfGfaaacaaaaaa | usUfsuUfuUfguuucacAfaAfcaagcsusg |
| SN-252172 | gsusuuguGfaAfAfCfaaaaaagugu | asCfsaCfuUfuuuuguuUfcAfcaaacsasa |
| SN-252173 | csasaaaaAfgUfGfUfucccuuuuca | usGfsaAfaAfgggaacaCfuUfuuuugsusu |
| SN-252174 | cscscuuuUfcAfAfGfuugagaacaa | usUfsgUfuCfucaacuuGfaAfaagggsusu |
| SN-252175 | gsasgaacAfaAfAfAfuuggguuuua | usAfsaAfaCfccaauuuUfuGfuucucsasa |
| SN-252176 | gsasacaaAfaAfUfUfggguuuuaaa | usUfsuAfaAfacccaauUfuUfuguucsusc |
| SN-252177 | gscsauugCfcUfUfCfgguuuguauu | asAfsuAfcAfaaccgaaGfgCfaaugcsasa |
| SN-252178 | csasuugcCfuUfCfGfguuuguauuu | asAfsaUfaCfaaaccgaAfgGfcaaugscsa |
| SN-252179 | cscsuucgGfuUfUfGfuauuuagugu | asCfsaCfuAfaauacaaAfcCfgaaggscsa |
| SN-252180 | gsgsuuugUfaUfUfUfagugucuuga | usCfsaAfgAfcacuaaaUfaCfaaaccsgsa |
| SN-252181 | gsusauuuAfgUfGfUfcuugaaugua | usAfscAfuUfcaagacaCfuAfaauacsusu |
| SN-252182 | gsusgucuUfgAfAfUfguaagaacau | asUfsgUfuCfuuacauuCfaAfgacacsusu |
| SN-252183 | gsuscuugAfaUfGfUfaagaacauga | usCfsaUfgUfucuuacaUfuCfaagacsasc |
| SN-252184 | cscsauagCfuGfGfUfuauuucuccu | asGfsgAfgAfaauaaccAfgCfuauggsusu |

In the table, a/c/g/u = 2'-OMe nucleotide; Af/Cf/Gf/Uf = 2'-F nucleotide; and s = phosphorothioate diester bond.

0.5 mL of cell culture medium (DMEM, 10% fetal bovine serum, and 1% penicillin-streptomycin solution) containing 10⁴ Hep3B cells (Procell, Cat# CL-0102) was added into a 96-well cell culture dish, and incubated overnight in a cell incubator with 5% CO₂ at 37°C. RNAiMAX (1.5 µL per well) and the small interfering nucleic acids (siRNAs) in Table 3 were added into Opti-MEM medium, and then the resulting mixture was added to the cell culture wells until a final concentration in each well reached 1 nM or 10 nM, and further cultured for 48 hours in a cell incubator with 5% CO₂ at 37°C. To extract RNA, the cell culture supernatant was aspirated completely, and the wells were rinsed with PBS. After removing residual liquid, 50 µL of prepared lysis buffer (as recommended in the Cells-to-CT Kit (Thermo Fisher Scientific, Cat#4391851c)) was added and mixed well. After 10 minutes of standing, 2.5 µL of Stop solution was added to terminate the reaction for 2 minutes. RT-PCR was performed as per the recommendation in the High Capacity cDNA Reverse Transcription Kits (Thermo Fisher, Catalog No.: 4368814), with 10 mL of lysed liquid in each reaction. Gene expression quantification was measured by using real-time fluorescent PCR. The TaqMan probe for human AGT was Hs00174854_m1, and the probe for the reference gene (human HPRT1) was Hs02800695_m1 (Thermo Fisher Scientific, Waltham, MA, USA). The PCR conditions were as follows: one cycle of 95°C for 20 seconds, followed by 40 cycles of 95°C for 1 second and 60°C for 20 seconds. The real-time fluorescent PCR instrument was the QuantStudio^{™} 6 Pro Real-Time PCR System (Thermo Fisher). The expression of the AGT gene was calculated using the 2^-ΔΔCt method, and the expression of the human HPRT1 gene was used as an internal reference. The expression level of the AGT gene is presented as a relative value compared with the cell group treated with RNAiMAX alone. The results are shown in Table 5.

**Table 5**

| Sample No. | 1nM | Standard deviation | 10nM | Standard deviation |
|---|---|---|---|---|
| SN-252158 | 1.82 | 0.09 | 0.22 | 0.09 |
| SN-252159 | 2.67 | 0.07 | 0.78 | 0.21 |
| SN-252160 | 0.41 | 0.06 | 0.29 | 0.09 |
| SN-252161 | 0.90 | 0.04 | 0.22 | 0.02 |
| SN-252163 | 0.90 | 0.03 | 0.15 | 0.07 |
| SN-252164 | 0.35 | 0.02 | 0.19 | 0.05 |
| SN-252165 | 0.89 | 0.06 | 0.15 | 0.07 |
| SN-252166 | 1.63 | 0.01 | 0.14 | 0.01 |
| SN-252167 | 0.91 | 0.12 | 0.18 | 0.03 |
| SN-252168 | 0.90 | 0.25 | 0.61 | 0.06 |
| SN-252169 | 0.92 | 0.06 | 0.60 | 0.01 |
| SN-252170 | 0.31 | 0.02 | 0.22 | 0.02 |
| SN-252171 | 2.43 | 0.46 | 0.49 | 0.01 |
| SN-252172 | 0.25 | 0.00 | 0.12 | 0.03 |
| SN-252173 | 0.40 | 0.03 | 0.16 | 0.02 |
| SN-252174 | 0.31 | 0.12 | 0.08 | 0.01 |
| SN-252175 | 0.38 | 0.09 | 0.23 | 0.03 |
| SN-252176 | 0.26 | 0.14 | 0.29 | 0.01 |
| SN-252177 | 1.67 | 0.84 | 1.11 | 0.07 |
| SN-252178 | 1.22 | 0.39 | 1.05 | 0.01 |
| SN-252179 | 1.49 | 0.44 | 1.53 | 0.07 |
| SN-252180 | 1.48 | 0.55 | 1.27 | 0.03 |
| SN-252181 | 1.11 | 0.05 | 0.69 | 0.12 |
| SN-252182 | 1.26 | 0.12 | 0.35 | 0.10 |
| SN-252183 | 1.68 | 0.05 | 0.94 | 0.24 |
| SN-252184 | 0.97 | 0.00 | 0.49 | 0.11 |

To further determine the activity of the siRNAs, a dose-response experiment was conducted in human primary hepatocytes using the siRNAs with superior efficacy listed in Table 5, which were conjugated to Tri-GalNAc (its specific structure is shown in Formula I above; the conjugated sequences are provided in Table 6). The siRNA-GalNAc samples were separately dissolved in 100 µL of enzyme-free and sterile water to prepare 100 µM solutions. 30 µL of the 100 µM test substance solution was added to 70 µL of inVitroGRO Plating Medium for dilution to prepare a 30 µM solution for later use as the working solution for the 30 nM final concentration group. Subsequently, the 30 µM test substance solution was diluted by 3 folds with inVitroGRO Plating Medium to achieve 8 concentration points, so that the final working solution concentrations were 4.6, 13.7, 41.2, 123.4, 370.4, 1111.1, 3333.3, and 10000 nM. Primary human hepatocytes were thawed from liquid nitrogen, recovered at 37°C, rinsed with serum-containing inVitroGRO Plating Medium, counted, and centrifuged. After removing the supernatant, the cells were resuspended in fresh serum-containing inVitroGRO Plating Medium at a concentration of 300k cells/mL. Then, 90 µL of the cell suspension was seeded into the wells of a 96-well cell culture plate, resulting in 30k cells per well. The prepared sample working solution was added to the cell suspension to achieve final concentrations of 0.46, 1.37, 4.12, 12.34, 37.04, 111.11, 333.33, and 1000 nM. Finally, the plate was placed in an incubator with 5% CO₂ and cultured at a constant temperature of 37 °C for 48 hours. 48 hours later, all the medium in the 96-well culture plate was aspirated, and the plate was washed with 1×PBS buffer. Then 50 µL of the prepared Cells-to-CT lysis buffer (as per the manufacturer's recommendations) was added and mixed thoroughly. After standing for 10 minutes, 2.5 µL of stop solution was added to terminate the reaction in 2 minutes. RT-PCR was performed as per the recommendation in the High Capacity cDNA Reverse Transcription Kits (Thermo Fisher, Catalog No.: 4368814), with 10 mL of lysed liquid in each reaction. Gene expression quantification was measured by using real-time fluorescent PCR. The TaqMan probe for human AGT was Hs00174854_m1, and the probe for the reference gene (human HPRT1) was Hs02800695_m1 (Thermo Fisher Scientific, Waltham, MA, USA). The PCR conditions were as follows: one cycle of 95°C for 20 seconds, followed by 40 cycles of 95°C for 1 second and 60°C for 20 seconds. The real-time fluorescent PCR instrument was the QuantStudio^{™} 6 Pro Real-Time PCR System (Thermo Fisher). The expression of the AGT gene was calculated using the 2^-ΔΔCt method, and the expression of the human HPRT1 gene was used as an internal reference. The expression level of the AGT gene was presented as a percentage relative to the control group treated with the culture medium alone, and the IC50 value was calculated accordingly (see Table 7).

**Table 6**

| No. | Sense strand (5'-3') | Antisense strand (5'-3') |
|---|---|---|
| SN-682160 | gsuscucaCfuUfUfCfcagcaaaacu-TriGalNAc | asGfsuUfuUfgcuggaaAfgUfgagacsusu |
| SN-682161 | cscsucaaCfuGfGfAfugaagaaacu-TriGalNAc | asGfsuUfuCfuucauccAfgUfugaggsgsa |
| SN-682163 | ususccugUfuUfGfCfuguguaugau-TriGalNAc | asUfscAfuAfcacagcaAfaCfaggaasusg |
| SN-682164 | gscsugggUfuUfAfUfuuuagagaau-TriGalNAc | asUfsuCfuCfuaaaauaAfaCfccagcsusu |
| SN-682165 | csusggguUfuAfUfUfuuagagaaug-TriGalNAc | csAfsuUfcUfcuaaaauAfaAfcccagsusu |
| SN-682170 | cscsagcuUfgUfUfUfgugaaacaaa-TriGalNAc | usUfsuGfuUfucacaaaCfaAfgcuggsusc |
| SN-682172 | gsusuuguGfaAfAfCfaaaaaagugu-TriGalNAc | asCfsaCfuUfuuuuguuUfcAfcaaacsasa |
| SN-682173 | csasaaaaAfgUfGfUfucccuuuuca-TriGalNAc | usGfsaAfaAfgggaacaCfuUfuuuugsusu |
| SN-682174 | cscscuuuUfcAfAfGfuugagaacaa-TriGalNAc | usUfsgUfuCfucaacuuGfaAfaagggsusu |
| SN-682175 | gsasgaacAfaAfAfAfuuggguuuua-TriGalNAc | usAfsaAfaCfccaauuuUfuGfuucucsasa |
| SN-682176 | gsasacaaAfaAfUfUfggguuuuaaa-TriGalNAc | usUfsuAfaAfacccaauUfuUfuguucsusc |

In the table, a/c/g/u = 2'-OMe nucleotide; Af/Cf/Gf/Uf = 2'-F nucleotide; and s = phosphorothioate diester bond.

**Table 7**

| No. | IC50 (nM) |
|---|---|
| SN-682160 | 296.8 |
| SN-682161 | 380.3 |
| SN-682163 | 505.8 |
| SN-682164 | 467.2 |
| SN-682165 | 701.0 |
| SN-682170 | 345.9 |
| SN-682172 | 253.4 |
| SN-682173 | 79.4 |
| SN-682174 | 344.5 |
| SN-682175 | 8.7 |
| SN-682176 | 72.2 |

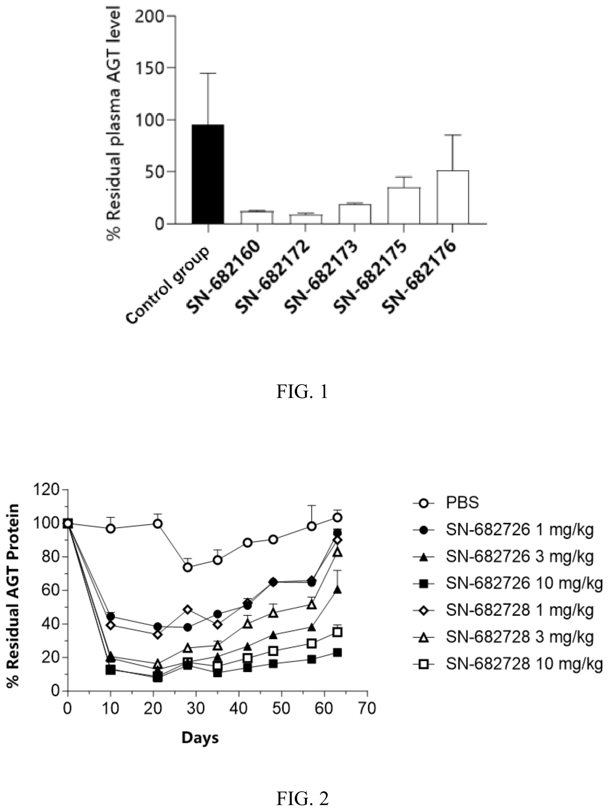

To further verify the efficacy of siRNA, SN-682160, SN-682172, SN-682173, SN-682175, and SN-682176 were each subcutaneously injected into human AGT transgenic mice at a dose of 10 mg/kg. On day 10, blood was sampled to detect the residual AGT protein level in plasma. The results are shown in FIG. 1. As can be seen from FIG. 1, on Day 10, SN-682172 can knock down the AGT protein level by 90% or more.

### Example 2 Optimization of siRNA Modification Methods

To further enhance the activity of siRNA, new modifications were made to the siRNA according to Table 8.

**Table 8**

| Number of siRNA | Sense strand | Antisense strand |
|---|---|---|
| SN-682725 | gsusuUfguGfaAfAfCfaaaaaagusu-TrigalNac | AfsCfsacuUfuuUfuguuUfcAfcaaacsasa |
| SN-682726 | gsusuUfguGfaAfAfCfaaaaaagugu-TrigalNac | AfsCfsacuuUfuUfuguuUfcAfcaaacsasa |
| SN-682727 | gsUfsuUfguGfaAfAfCfaaaaaagugu-TrigalNac | AfsCfsacuUfuuUfuguuUfcAfcaaacsasa |
| SN-682728 | gsUfsuUfguGfaAfAfCfaaaaaagugu-TrigalNac | AfsCfsacuuUfuUfuguuUfcAfcaaacsasa |
| SN-682172 | gsusuuguGfaAfAfCfaaaaaagugu-TrigalNac | asCfsaCfuUfuuuuguuUfcAfcaaacsasa |

In the table, a/c/g/u = 2'-OMe nucleotide; Af/Cf/Gf/Uf = 2'-F nucleotide; and s = phosphorothioate diester bond.

To further determine the activity of the siRNAs, a dose-response experiment was conducted with the siRNAs listed in Table 8 in human primary hepatocytes, following the same method as described above. The absolute IC50 values for reducing AGT expression are provided in Table 9.

**Table 9**

| Sample No. | IC50 (nM) |
|---|---|
| SN-682725 | 70.20 |
| SN-682726 | 8.60 |
| SN-682727 | 44.10 |
| SN-682728 | 21.80 |
| SN-682172 | 375.40 |

### Example 3 In Vitro Efficacy Verification of siRNA

To further verify the efficacy, the preferred siRNA drugs were compared with SN-2073 in human primary hepatocytes, and the involved siRNAs are listed in Table 10. Among them, SN-2073 used as the control was prepared in accordance with the disclosure of US11015201B2. The experimental procedures in human primary hepatocytes were similar to the aforementioned experimental methods. The expression level of the AGT gene is presented as a percentage relative to the control group of cells treated with vehicle alone, and the results are shown in Table 11.

**Table 10**

| Number of siRNA | Sense strand | Antisense strand |
|---|---|---|
| SN-682726 | gsusuUfguGfaAfAfCfaaaaaagugu-TrigalNac | AfsCfsacuuUfuUfuguuUfcAfcaaacsasa |
| SN-682728 | gsUfsuUfguGfaAfAfCfaaaaaagugu-TrigalNac | AfsCfsacuuUfuUfuguuUfcAfcaaacsasa |
| SN-2073 | gsuscaucCfaCfAfAfugagaguaca- TrigalNac | usGfsuacTgncucauugUfgGfaugacsgsa |

In the table: a/c/g/u = 2'-O-methyl nucleotide; Af/Cf/Gf/Uf = 2'-fluoro nucleotide. Tgn = thymidine glycol; and s = phosphorothioate diester bond. Structures used for SN-682726 and SN-682728 are shown in Formula I, and Tri-GalNAc used for SN-2073 is Alnylam Tri-GalNAc (L96). Both the structure shown in Formula I and L96 are classified as triantennary N-acetylgalactosamine. Therefore, based on the following efficacy data, the advantages of the modified sequences in the present disclosure can also be confirmed.

**Table 11**

| Sample No. | 1000 nM Mean | Standard deviation | 100 nM | Standard deviation |
|---|---|---|---|---|
| SN-682726 | 16.25 | 0.99 | 25.27 | 2.77 |
| SN-682728 | 21.80 | 0.74 | 33.30 | 4.61 |
| SN-2073 | 36.00 | 0.62 | 33.63 | 3.07 |

It can be learned from the free uptake experiment in primary human hepatocytes that SN-682726 and SN-682728 in the present disclosure exhibit better activity than SN-2073.

### Example 4 In Vivo Efficacy Verification of siRNA

To further verify the activity of siRNA, human AGT transgenic mice were subcutaneously injected with 1, 3, and 10 mg/kg of SN-682726 or SN-682728 on Day 0, respectively, and PBS was used as the blank control group. The human AGT protein in the blood was then monitored. The results are shown in FIG. 2.

As can be seen from FIG. 2, both SN-682726 and SN-682728 can significantly reduce the level of AGT protein and exhibit efficacy for a longer time. SN-682726 shows better efficacy.

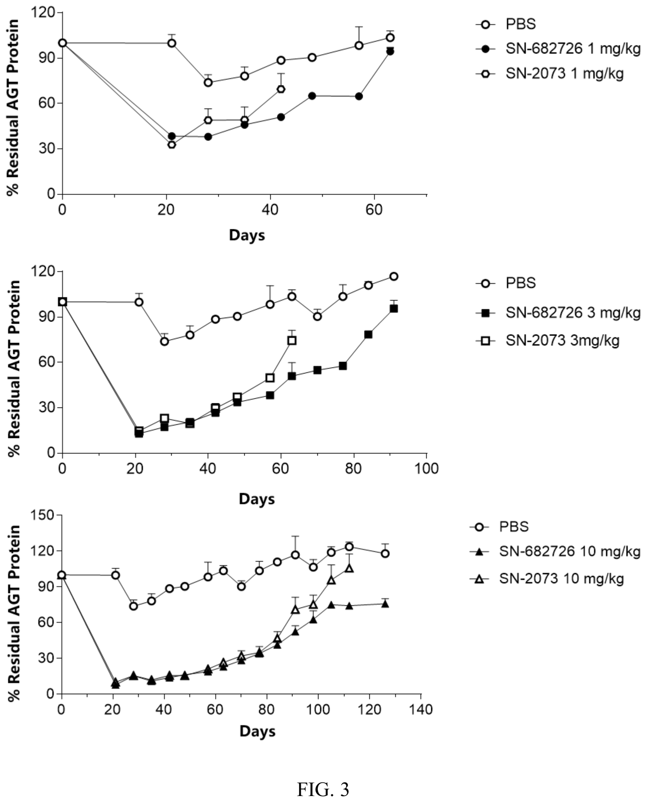

To further verify the effect of modification on efficacy, SN-682726 was compared with SN-2073 in human AGT transgenic mice. The mice were subcutaneously injected with 1, 3, and 10 mg/kg of compound SN-682726 or SN-2073 on Day 0, respectively, and PBS was used as the blank control group. The human AGT protein in the blood was then monitored. The results are shown in FIG. 3.

As can be seen from FIG. 3, SN-682726 can maintain efficacy for a longer time than the reference compound SN-2073 in all dose groups, and still exhibited a 40% knockdown effect on Day 130 after injection, while the AGT protein level in the control group receiving SN-2073 had returned to the baseline on Day 110 after injection, demonstrating the superiority of SN-682726.

In addition, it should be noted that the efficacy advantages of the siRNA drug in the present disclosure do not depend on the selection of the targeting carrier. After the TriGalNAc is replaced with other applicable carriers (for example, L96 as described above), the resulting siRNA drugs still exhibit the foregoing significant efficacy advantages compared with SN-2073 in both *in vitro* cell experiments and *in vivo* experiments. For example, after the TriGalNAc of SN-682726 is replaced with L96, the drug still exhibits a better AGT expression silencing effect than SN-2073 in primary monkey hepatocytes, as shown in Table 12.

**Table 12**

| siRNA | 1000 nM | Standard deviation | 250 nM | Standard deviation |
|---|---|---|---|---|
| SN-682726-L96 | 74.5% | 0.5% | 74.7% | 4.0% |
| SN-2073-L96 | 66.2% | 6.0% | 69.8% | 4.4% |

The modification of SN-682726 was also made to SN-2073, to obtain SN-683015 shown in Table 13. It can be seen that SN-683015 exhibited better efficacy than SN-2073 in primary monkey hepatocytes, and the results are shown in Table 14.

**Table 13**

| No. | Sense strand | Antisense strand |
|---|---|---|
| SN-683015 | gsuscAfucCfaCfAfAfugagaguaca-L96 | UfsGfsuacuCfuCfauugUfgGfaugacsgsa |
| SN-2073 | gsuscaucCfaCfAfAfugagaguaca-L96 | usGfsuacTgncucauugUfgGfaugacsgsa |

**Table 14**

| siRNA | 1000 nM | Standard deviation | 250 nM | Standard deviation |
|---|---|---|---|---|
| SN-683015 | 82.2% | 3.6% | 83.0% | 2.8% |
| SN-2073 | 66.2% | 6.0% | 69.8% | 4.4% |

In addition, an unmodified sequence (SN-122726) of SN-682726 and an unmodified sequence (SN-122073 in Table 15) of SN-2073 at three concentrations (0.1, 1, and 10 nM) were also compared in Hep3B cells. The results are shown in Table 16. It can be seen that the unmodified SN-122726 in the present disclosure can more effectively reduce the AGT expression level.

**Table 15**

| No. | Sense strand | Antisense strand |
|---|---|---|
| SN-122726 | GUUUGUGAAACAAAAAAGUGU | ACACUUUUUUGUUUCACAAACAA |
| SN-122073 | GUCAUCCACAAUGAGAGUACA | UGUACUCUCAUUGUGGAUGACGA |

**Table 16**

| siRNA | 0.1nM | Standard deviation | 1nM | Standard deviation | 10nM | Standard deviation |
|---|---|---|---|---|---|---|
| SN-122726 | 92.7% | 4.3% | 97.3% | 0.1% | 98.5% | 0.2% |
| SN-122073 | 82.8% | 0.5% | 91.6% | 1.1% | 96.5% | 0.6% |

The above examples represent only a few embodiments of the present disclosure. Although described in detail, they should not be construed as limiting the scope of the present disclosure. It should be noted that those skilled in the art may make various modifications and improvements without departing from the spirit of the present disclosure, and such variations are within the scope of protection of the present disclosure.

## Claims

1. A nucleic acid comprising a sense strand and an antisense strand, wherein the sense strand comprises a sequence having at least 80% sequence identity with a sequence as set forth in any one of SEQ ID NOs: 1 to 26, and the antisense strand comprises a sequence having at least 80% sequence identity with a sequence as set forth in any one of SEQ ID NOs: 27 to 52.

2. The nucleic acid according to claim 1, wherein the antisense strand comprises at least 15 consecutive nucleotides that differ from a sequence as set forth in any one of SEQ ID NOs: 27 to 52 by no more than 3 nucleotides, and the sense strand comprises a nucleotide sequence that is at least partially complementary to the antisense strand; and optionally, the sense strand comprises at least 15 consecutive nucleotides that differ from a sequence as set forth in any one of SEQ ID NOs: 1 to 26 by no more than 3 nucleotides.

3. The nucleic acid according to claim 1, wherein the antisense strand comprises a nucleotide sequence that differs from a sequence as set forth in any one of SEQ ID NO: 29, SEQ ID NO: 32, SEQ ID NO: 38, SEQ ID NO: 40, SEQ ID NO: 41, SEQ ID NO: 42, SEQ ID NO: 43, or SEQ ID NO: 44 by 0, 1, or 2 nucleotides, and the sense strand comprises a nucleotide sequence that is at least partially complementary to the antisense strand; and optionally, the sense strand comprises a nucleotide sequence that differs from a sequence as set forth in any one of SEQ ID NO: 3, SEQ ID NO: 6, SEQ ID NO: 12, SEQ ID NO: 14, SEQ ID NO: 15, SEQ ID NO: 16, SEQ ID NO: 17, or SEQ ID NO: 18 by 0, 1, or 2 nucleotides.

4. The nucleic acid according to claim 1, wherein the antisense strand comprises a nucleotide sequence that differs from the sequence as set forth in any one of SEQ ID NO: 29, SEQ ID NO: 40, SEQ ID NO: 41, SEQ ID NO: 43, or SEQ ID NO: 44 by 0, 1, or 2 nucleotides, and the sense strand comprises a nucleotide sequence that is at least partially complementary to the antisense strand; and optionally, the sense strand comprises a nucleotide sequence that differs from the sequence as set forth in any one of SEQ ID NO: 3, SEQ ID NO: 14, SEQ ID NO: 15, SEQ ID NO: 17, or SEQ ID NO: 18 by 0, 1, or 2 nucleotides.

5. The nucleic acid according to claim 1, wherein the antisense strand comprises a nucleotide sequence that differs from the sequence as set forth in SEQ ID NO: 40 by 0, 1, or 2 nucleotides, and the sense strand comprises a nucleotide sequence that is at least partially complementary to the antisense strand; and optionally, the sense strand comprises a nucleotide sequence that differs from the sequence as set forth in SEQ ID NO: 14 by 0, 1, or 2 nucleotides.

6. The nucleic acid according to claim 1, wherein a content of GC in the nucleic acid is 20%-45%, preferably 20%-35%.

7. The nucleic acid according to claim 1, wherein at least one nucleotide in the nucleic acid is a modified nucleotide or comprises a modified internucleotide linkage;
the modified nucleotide is preferably one or more selected from the group consisting of 2'-O-methyl nucleotide, 2'-fluoro nucleotide, 2'-deoxy nucleotide, 2',3'-seco nucleotide mimetic, locked nucleotide, 2'-F-arabinonucleotide, 2'-methoxyethyl nucleotide, abasic nucleotide, ribitol, inverted nucleotide, inverted 2'-O-methyl nucleotide, inverted 2'-deoxy nucleotide, 2'-amino modified nucleotide, 2'-alkyl modified nucleotide, morpholino nucleotide, nucleotide containing vinyl phosphonate, nucleotide containing cyclopropyl phosphonate, and 3'-O-methyl nucleotide; and the modified nucleotide is more preferably one or more selected from the group consisting of 2'-O-methyl nucleotide and 2'-fluoro nucleotide; and
the modified internucleotide linkage is preferably one or more selected from the group consisting of phosphorothioate internucleotide linkage and methylphosphonate internucleotide linkage; and the modified internucleotide linkage is more preferably one or more selected from the group consisting of phosphorothioate monoester internucleotide linkage and phosphorothioate diester internucleotide linkage.

8. The nucleic acid according to claim 7, wherein, counted from the 5' end of the antisense strand, nucleotides at positions 5 and 6 are 2'-O-methyl nucleotides, and the nucleotide at position 7 is a 2'-fluoro nucleotide; and
preferably, each of the 5' end and 3' end of the antisense strand contains two phosphorothioate internucleotide linkages, nucleotides at positions 1, 2, 7, 9, 14, and 16, counted from the 5' end, are 2'-fluoro nucleotides, and all of the remaining nucleotides are 2'-O-methyl nucleotides.

9. The nucleic acid according to claim 7 or 8, wherein the nucleotide at position 2, counted from the 5' end of the sense strand, is a 2'-O-methyl nucleotide or a 2'-fluoro nucleotide, preferably a 2'-O-methyl nucleotide; and
preferably, the 5' end of the sense strand contains two phosphorothioate internucleotide linkages, nucleotides at positions 4, 7, 9, 10, and 11, counted from the 5' end, are 2'-fluoro nucleotides, and all of the remaining nucleotides are 2'-O-methyl nucleotides.

10. The nucleic acid according to claim 1, wherein the antisense strand has at least 15 consecutive nucleotides that differ from any one of antisense strand sequences shown in Table 3 or Table 8 by no more than 3 nucleotides, the sense strand has at least 15 consecutive nucleotides that differ from any one of sense strand sequences shown in Table 3 or Table 8 by no more than 3 nucleotides; and preferably, the sense strand and the antisense strand form any one of siRNAs shown in Table 4 or Table 8.

11. The nucleic acid according to claim 1, wherein the antisense strand comprises, from the 5' end to the 3' end, a nucleotide sequence that differs from the following nucleotide sequence by 0, 1, or 2 nucleotides:
AfsCfsacuuUfuUfuguuUfcAfcaaacsasa;
the sense strand comprises a nucleotide sequence that is at least partially complementary to the antisense strand; and
preferably, the sense strand comprises, from the 5' end to the 3' end, a nucleotide sequence that differs from any one of the following nucleotide sequences by 0, 1, or 2 nucleotides:
gsusuUfguGfaAfAfCfaaaaaagugu; or
gsUfsuUfguGfaAfAfCfaaaaaagugu;
wherein in each sequence, a nucleotide represented by a lowercase letter is 2'-O-methyl nucleotide; "f" indicates that an adjacent nucleotide on the left side is a nucleotide modified by 2'-fluoro; and "s" indicates that two adjacent nucleotides on the left and right sides are linked by a phosphorothioate diester bond.

12. A targeted drug delivery system, wherein the targeted drug delivery system comprises a targeting moiety, a linking moiety, and the nucleic acid according to any one of claims 1 to 11 that is linked to the targeting moiety through the linking moiety.

13. The targeted drug delivery system according to claim 12, wherein the targeted drug delivery system comprises a ligand and the nucleic acid linked to the ligand; preferably, the ligand is a GalNAc derivative; more preferably, the ligand is one or more GalNAc derivatives linked via a single-stranded, double-stranded or triple-stranded branched linker; and even more preferably, a structure of the targeted drug delivery system is represented by a formula of 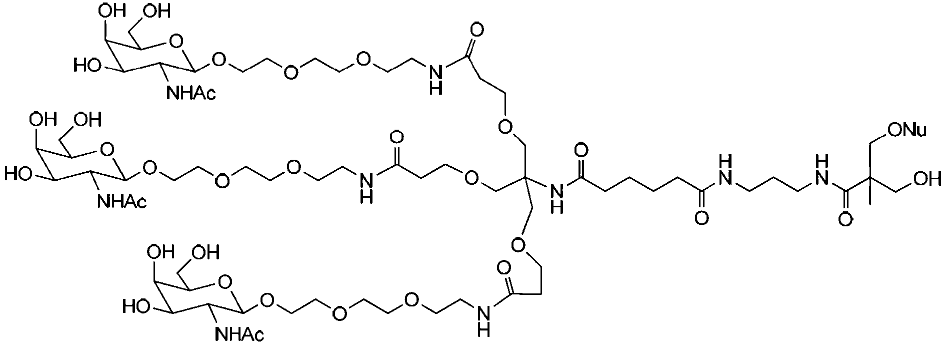 in which Nu represents the nucleic acid.

14. A pharmaceutical composition, wherein the pharmaceutical composition comprises the nucleic acid according to any one of claims 1 to 11, or the targeted drug delivery system according to any one of claims 12 to 13, and a pharmaceutically acceptable carrier.

15. A method of inhibiting expression of angiotensin gene in a cell, the method comprising: contacting the cell with the nucleic acid according to any one of claims 1 to 11, the targeted drug delivery system according to any one of claims 12 to 13, or the pharmaceutical composition according to claim 14, to inhibit the expression of the angiotensin gene in the cell.

16. Use of the nucleic acid according to any one of claims 1 to 11, the targeted drug delivery system according to any one of claims 12 to 13, or the pharmaceutical composition according to claim 14 in any one of the following aspects:
1) treatment and/or prevention of a disease associated with angiotensin; or
2) preparation of a medicament for treating and/or preventing a disease associated with angiotensin.

17. The use according to claim 16, wherein the disease is:
i) a disease associated with enhancement or elevation of an activity level of angiotensin; or
ii) a disease that benefits from reduced expression of angiotensinogen.

18. The use according to claim 16, wherein the disease is a disease associated with excessive activation of a renin-angiotensin-aldosterone system.

19. The use according to claim 16, wherein the disease is one or more selected from the group consisting of hypertension, hypertensive disorder, borderline hypertensive disorder, primary hypertensive disorder, secondary hypertensive disorder, isolated systolic or diastolic hypertensive disorder, pregnancy-related hypertensive disorder, diabetic hypertensive disorder, resistant hypertensive disorder, refractory hypertensive disorder, paroxysmal hypertensive disorder, renovascular hypertensive disorder, Goldblatt hypertension, hypertensive disorder associated with low plasma renin activity or plasma renin concentration, high intraocular pressure, glaucoma, pulmonary arterial hypertension, portal hypertension, systemic venous hypertension, systolic hypertensive disorder, unstable hypertensive disorder; hypertensive heart disease, hypertensive nephropathy, atherosclerosis, arteriosclerosis, vascular disease, diabetic nephropathy, diabetic retinopathy, chronic heart failure, cardiomyopathy, diabetic cardiomyopathy, glomerulosclerosis, coarctation of the aorta, aortic aneurysm, ventricular fibrosis, heart failure, myocardial infarction, angina pectoris, stroke, nephropathy, renal failure, systemic sclerosis, intrauterine growth restriction, fetal growth restriction, obesity, hepatic steatosis/fatty liver, non-alcoholic steatohepatitis, non-alcoholic fatty liver disease, glucose intolerance, type 2 diabetes mellitus, and metabolic syndrome.

20. The use according to claim 16, wherein the nucleic acid, the targeted drug delivery system, or the pharmaceutical composition is administered to a subject via subcutaneous administration, intravenous administration, and/or intramuscular administration.
